(19) Europäisches Patentamt / European Patent Office / Office européen des brevets

(11) **EP 4 546 369 A1**

(12) **EUROPEAN PATENT APPLICATION**

(43) Date of publication:
30.04.2025 Bulletin 2025/18

(21) Application number: 24208665.0

(22) Date of filing: 24.10.2024

(51) International Patent Classification (IPC):
**G16H 50/30** (2018.01)    **G16H 50/70** (2018.01)

(52) Cooperative Patent Classification (CPC):
**G16H 50/30; G16H 50/70**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: 27.10.2023 US 202363593736 P

(71) Applicant: **Insulet Corporation**
**Acton, MA 01720 (US)**

(72) Inventors:
• **NARAYANASWAMI, Rangarajan**
**Weston, MA 02493 (US)**
• **KODUKULA, Satya**
**San Diego, CA 92129 (US)**

(74) Representative: **Peterreins Schley**
**Patent- und Rechtsanwälte PartG mbB**
**Hermann-Sack-Straße 3**
**80331 München (DE)**

(54) **AUTOMATED MEDICAMENT DELIVERY DEVICES, CONTROLLERS, AND METHODS FOR PATIENT PROTECTION, MANAGEMENT, AND COMMUNICATION**

(57)     Systems and methods for patient protection, management, and communication are disclosed. The methods include utilizing data captured by the automated medicament delivery device to detect a fall of the patient and analyze a post fall recovery of the patient, receiving inputs and commands at the automated medicament delivery device, providing alerts at the automated medicament delivery device, processing alerts triggered at the automated medicament delivery device, triggering alerts at the automated medicament delivery device, and customizing alerts provided at the automated medicament delivery device.

FIG. 4

EP 4 546 369 A1

## Description

TECHNICAL FIELD

The present disclosure generally relates to medicament delivery systems. More particularly, the present disclosure relates to automated medicament delivery devices, controllers, and methods for patient protection, management, and communication.

BACKGROUND

Automated medicament delivery devices (e.g., Automated Insulin Delivery (AID) device, without limitation) are often used to administer medicaments to the body of a patient via a cannula inserted into the body to treat medical conditions. The medical conditions may increase the risk of falls. Further, some medicaments (e.g., blood pressure medicaments taken for hypertension that may cause postural hypotension) may increase risk of falls. A caregiver of the patient (e.g., parent, nurse, child, without limitation) or other interested party may not always be next to the patient and may need to be alerted in the event of a fall.

Further, a caregiver may want to know about a patient's current condition, current medicament administration, or other emergency related to the patient's medical condition and to monitor and be alerted to certain conditions and emergencies.

Patients may also not always have the controller for an automated medicament delivery device (e.g., a dedicated controller, mobile device, without limitation) in audible or visual range and may not notice alerts or messages provided by the controller or messages sent to the controller by the caregiver.

Further, patients may misplace or lose the controller, or device used to communicate with the controller, associated with the automated medicament delivery device, which may inhibit adjusting or controlling medicament deliveries to the body otherwise managed via the controller.

DISCLOSURE

In one illustrative embodiment, the present disclosure provides a method. The method includes obtaining motion data by sampling the motion sensor of an automated medicament delivery device at a predetermined frequency. The method also includes determining a magnitude of resultant values using the motion data. The method further includes storing the resultant values in in a buffer for a time window in the memory. The method also includes determining a difference between a maximum value and a minimum value of the resultant values currently stored in the buffer. The method also includes comparing the difference to a fall threshold value. The method further includes setting a fall indication at least partially responsive to the comparison.

In another illustrative embodiment, the present disclosure provides an automated medicament delivery device. The automated medicament delivery device includes a motion sensor, one or more processors, and memory. The memory includes instructions, when executed, cause the one or more processors to: obtain motion data by sampling the motion sensor at a predetermined frequency; determine a magnitude of resultant values using the motion data; store the resultant values in in a buffer for a time window in the memory; determine a difference between a maximum value and a minimum value of the resultant values currently stored in the buffer; compare the difference to a fall threshold value; and in response to the difference being greater than the fall threshold value, indicate that a fall has been detected.

In a further illustrative embodiment, the present disclosure provides a method. The method includes detecting a first tap at a motion sensor of an automated medicament delivery device. The method also includes listening for one or more tap sequences in response to detecting the first tap. The method further includes initiating, at the automated medicament delivery device, an action in response to recognition of a tap sequence associated with the action.

In another illustrative embodiment, the present disclosure provides an automated medicament delivery device. The automated medicament delivery device includes a motion sensor, one or more processors, and memory. The memory includes instructions, when executed, cause the one or more processors to: detect a first tap at the motion sensor; listen for one or more tap sequences in response to detecting the first tap; and initiate an action in response to recognition of a tap sequence associated with the action.

In another illustrative embodiment, the present disclosure provides a method. The method includes receiving, at a cloud server, an indication that an alarm has been triggered at an automated medicament delivery device. The method also includes notifying a caregiver that the alarm was triggered.

In another illustrative embodiment, the present disclosure provides a cloud server. The cloud server includes one or more processors and memory. The memory includes instructions, when executed, cause the one or more processors to: receive an indication that an alarm has been triggered at an automated medicament delivery device; and notify a caregiver that the alarm was triggered.

In another illustrative embodiment, the present disclosure provides a method. The method includes receiving, at a handheld electronic computing device associated with an automated medicament delivery device, an indication that an alarm has been triggered at the automated medicament delivery device. The method also includes obtaining geographic coordinates associated with the alarm. The method further includes obtaining geographic coordinates associated with the alarm. The method also includes sending the indication that the alarm has been triggered and the geographic coordinates to a cloud server.

In another illustrative embodiment, the present disclosure provides a handheld electronic computing device associated with an automated medicament delivery device. The handheld electronic computing device includes one or more processors and memory. The memory includes instructions, when executed, cause the one or more processors to: receive an indication that an alarm has been triggered at an automated medicament delivery device; obtain geographic coordinates associated with the alarm; and send the indication that the alarm has been triggered and the geographic coordinates to a cloud server.

In another illustrative embodiment, the present disclosure provides a process. The process includes obtaining motion data by sampling a motion sensor of an automated medicament delivery device. The process also includes determining a magnitude of dynamic motion data by subtracting a value of gravity from the motion data. The process further includes determining a signal energy from the magnitude of dynamic motion data. The process also includes determining whether an activity of the patient has occurred based at least in part by comparing the signal energy to an activity threshold value. The process further includes triggering an activity alert in response to a determination that the activity of the patient has occurred.

In another illustrative embodiment, the present disclosure provides a device. The device includes one or more processors and memory. The memory includes instructions, when executed, cause the one or more processors to: obtain motion data by sampling a motion sensor of an automated medicament delivery device; determine a magnitude of dynamic motion data by subtracting a value of gravity from the motion data; determine whether an activity of the patient has occurred based at least in part by comparing the signal energy to an activity threshold value; and trigger an activity alert in response to a determination that the activity of the patient has occurred.

In another illustrative embodiment, the present disclosure provides a method. The method includes selecting an alert for implementation on an automated medicament delivery device. The method also includes setting a time for the alert. The method further includes selecting an alert type. The method also includes sending instructions to the automated medicament delivery device for implementation of the alert thereon.

In another illustrative embodiment, the present disclosure provides a handheld electronic computing device associated with an automated medicament delivery device. The handheld electronic computing device includes one or more processors and memory. The memory includes instructions, when executed, cause the one or more processors to: select an alert for implementation on the automated medicament delivery device; setting a time for the alert; selecting an alert type; and sending instructions to the automated medicament delivery device for implementation of the alert thereon.

BRIEF DESCRIPTION OF THE DRAWINGS

The present disclosure is illustrated and described herein with reference to the various drawings, in which like reference numbers are used to denote like system components/method steps, as appropriate, and in which:

FIG. 1 is a schematic diagram illustrating a medicament delivery system in accordance with embodiments;
FIG. 2 is a block diagram of a medicament delivery system 200 for controlled administering of medicament, in accordance with one or more examples;
FIG. 3 is an example graph of acceleration over time determined from motion data obtained by the motion sensor of the automated medicament delivery device in accordance with one or more embodiments;
FIG. 4 is a flowchart of a method for detecting a fall of a patient in accordance with one or more embodiments;
FIG. 5 is a flowchart of a method for post fall analysis of a patient in accordance with one or more embodiments;
FIG. 6 is a flowchart of a method for receiving commands at an automated medicament delivery device in accordance with one or more embodiments;
FIG. 7 is a flowchart of a method for providing an alert in accordance with one or more embodiments;
FIG. 8 is a flowchart of a method for processing an alert in accordance with one or more embodiments;
FIG. 9 is a process 900 for triggering an alert in response to an identified activity of a patient in accordance with one or more embodiments; and
FIG. 10 is a flowchart of a method for customizing alerts provided at an automated medicament delivery device, in accordance with one or more embodiments.

MODE(S) FOR CARRYING OUT THE INVENTION

In various embodiments, medicament delivery systems, and in particular, automated medicament delivery devices and controllers, and methods for patient monitoring, protection, management, and communication utilizing the automated medicament delivery device are disclosed.

As will be described in detail below, the methods may include utilizing data captured by the automated medicament delivery device to detect a fall of the patient and analyze a post fall recovery of the patient, receiving inputs and commands at the automated medicament delivery device, providing alerts at the automated medicament delivery device, processing alerts triggered at the automated medicament delivery device, triggering alerts at the automated medicament delivery device, and customizing alerts provided at the automated medicament delivery device.

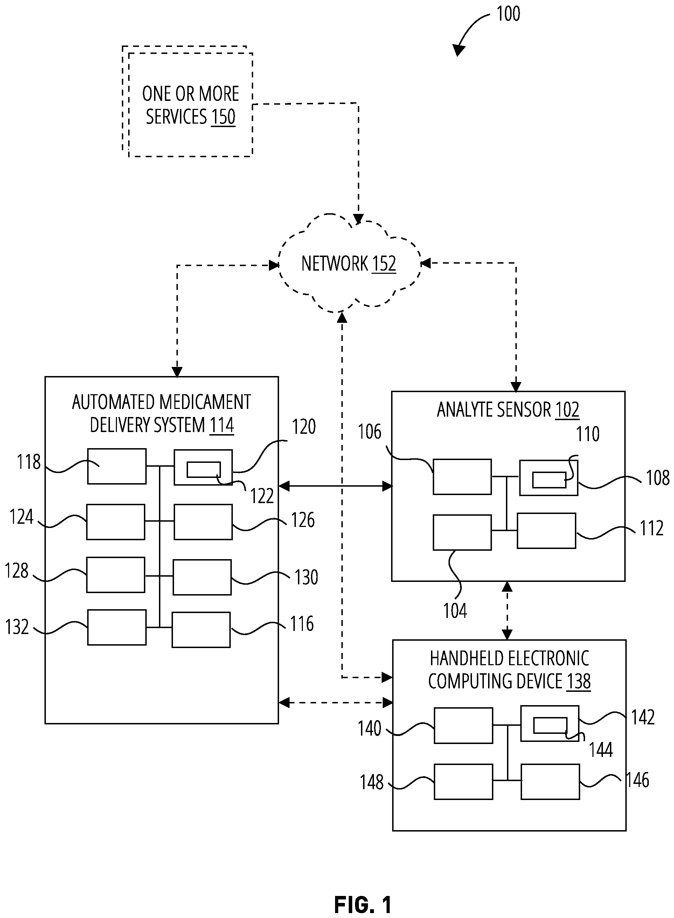

FIG. 1 is a schematic diagram illustrating a system 100 for automated administration of medicament to a user-body, in accordance with one or more embodiments.

In one or more embodiments, system 100 may be capable of one or more operative modes of administration of medicament. Non-limiting examples of the one or more operative modes include: fully automated administration of medicament, partially automated administration of medicament, or manual administration of medicament. In one or more embodiments, system 100 may be capable of alternating between multiple (e.g., two or more, without limitation) operative modes. As a non-limiting example, system 100 may alternate between one or more of: fully automated operation, partially automated operation, and manual operation.

System 100 may administer medicament at least partially based on one or more values representative of amounts of one or more analytes present within a user-body (such values respectively an "analyte value"). The one or more analytes may include constituents of the user-body and foreign substances, such as medicaments, markers, metabolites, and combinations or subcombinations of one or more of the foregoing, without limitation.

Non-limiting examples of medicaments administrable by system 100 include: insulin, glucagon-like peptide-1 receptor agonist (GLP-1), glucose-dependent insulinotropic polypeptide (GIP), or other hormones, insulin substitutes, and combinations of medicaments, such as two or more of insulin, GLP-1, and GIP, or other like hormones. While specific examples discussed herein may involve insulin or GLP-1, or GIP, this disclosure is not limited to those examples, and other medicaments do not exceed the scope. As a non-limiting example, glucagon, morphine, analgesics, fertility medicaments, blood pressure medicaments, chemotherapy drugs, arthritis drugs, weight loss drugs, without limitation are non-limiting examples of medicaments that are specifically contemplated, system 100.

System 100 includes an analyte sensor 102, automated medicament delivery device 114. System 100 may optionally include a handheld electronic computing device 138, network 152, and one or more services 150.

The analyte sensor 102 is configured to obtain data related to one or more analytes within the user-body ("analyte data"). In various embodiments, the analyte data may include one or more analyte values. In various embodiments, the analyte sensor 102 is an analytical biosensing device, such as a continuous glucose monitor (CGM) or an integrated continuous glucose monitor (ICGM) (e.g., examples of commercially available analytical bio-sensing devices include the FREESTYLE LIBRE® 3 manufactured by Abbott or the DEXCOM® G6 manufactured by Dexcom, without limitation).

The analyte sensor 102 includes a filament 104 and various electronic components. The filament 104 is configured to obtain data related to one or more analytes within a user-body and provide the data to the various electronic components of the analyte sensor 102. The filament 104 may be configured to obtain the data directly from fluids of a user-body, including without limitation interstitial fluids of a user-body, from tissue of a user-body, combinations thereof, or in any other manner known in the art.

The various electronic components of analyte sensor 102 include one or more processors 106, a memory 108, and a communication equipment 112. Instructions 110 include instructions for processing data obtained via filament 104. When instructions 110 are executed by one or more processors 106, the instructions 110 cause one or more processors 106 to process the data obtained via filament 104. The instructions 122 may be implemented in hardware (e.g., one or more hardware processors of one or more processors 106 such as an integrated circuit, application specific integrated circuit (ASIC), digital signal processor (DSP), or other logic circuit, without limitation), implemented in software (e.g., firmware, software, machine code, applications, without limitation), or a combination thereof. The instructions for processing the data obtained via filament 104 may include one or more instructions respectively for determining analyte values at least partially based on the data, or for sending the data, analyte values or both to automated medicament delivery device 114 or the one or more services 150.

The communication equipment 112 is configured to facilitate communication (e.g., a device or interface for wired communication, wireless communication, both wired and wireless communication, without limitation) of the analyte sensor 102 with other devices, including the automated medicament delivery device 114, without limitation. Such communication may be according to any appropriate wired or wireless communication protocol, such as Wi-Fi®, BLUETOOTH®, near-field communication (NFC), radio-frequency identification (RFID), or any other radio-frequency, infrared, or optical communication technology.

The automated medicament delivery device 114 is configured to administer medicament to a user-body, such as subcutaneously into the user-body, without limitation, in accordance with one or more embodiments. In one or more embodiments, automated medicament delivery device 114 may offer one or more operative modes for administration of medicament to a user-body. When operating in some of the operative modes, automated medicament delivery device 114 may administer medicament at least partially responsive to analyte values, including without limitation analyte values received from analyte sensor 102. When operating in some further operative modes, automated medicament delivery device 114 may administer medicament at least partially responsive to user input. When operating some yet further operative modes, automated medicament delivery device 114 may administer medicament at least partially responsive to analyte values and user input. Non-limiting examples of the one or more operative modes offered by automated medicament delivery device 114 include: fully automated administration of medicament, partially automated administration of medicament, or manual administration of medicament. When operating in an operative mode that includes manual administration of medicament, automated medicament delivery device 114 may administer medicament solely in response to a user input (e.g., delivers medicament in response to a user confirmation of delivery of medicament or in response to a user instruction to delivery medicament, without limitation). When operating in an operative mode that includes fully automated administration of medicament, automated medicament delivery device 114 may administer medicament solely in response to analyte values (e.g., delivers medicament in response to one or more analyte values, without limitation). When operating in an operative mode that includes partially automated administration of medicament, automated medicament delivery device 114 may administer medicament in response to analyte values and user input (e.g., delivers medicament in response to a user input and an analyte value, or alternately delivers medicament in response to a user input or in response to analyte values, without limitation).

The automated medicament delivery device 114 includes delivery system 116, one or more processors 118, memory 120, a communication equipment 124, a power source 126, and one or more sensors 128. In one or more embodiments, the automated medicament delivery device 114, or portions thereof, may be a wearable device and may be secured to a user-body (e.g., secured via one or more adhesive layers attaching the automated medicament delivery device 114 to the skin of the user-body or a material that is secured to the user body, without limitation).

In various embodiments, the delivery system 116 is configured to cause an amount of medicament to move (e.g., flow, without limitation) toward and/or into a user-body.

In various embodiments, delivery system 116 may deliver amounts of medicament at least partially responsive to requests. In various embodiments, instructions 122 of memory 120 may include instructions for determining and generating requests for delivery system 116. In various embodiments, instructions 122 may include instructions for determining one or more amounts of medicament, determining a timing for delivery of one or more amounts of medicament, and for generating one or more requests for delivery system 116 related to the same. When such instructions of instructions 122 are executed by one or more processors 118, the one or more processors 118 determine the amounts of medicament and timing of delivery, generate requests for the delivery system 116 at least partially based on the determined amounts and timing, and provide the requests to delivery system 116.

The communication equipment 124 is configured to facilitate communication (e.g., wireless communication, without limitation) of the automated medicament delivery device 114 with other devices, including without limitation communication between analyte sensor 102 and the automated medicament delivery device 114. The communication may be wired or wireless communication and may utilize any suitable communication protocol such as wireless networking protocol (e.g., Wi-Fi®, without limitation), a short-range wireless protocol (e.g., BLUETOOTH®, without limitation), a near-field communication standard, a cellular standard, or any other wireless optical or radio-frequency protocol. In various embodiments, the communication equipment 124 includes an Internet of Things (IOT) Subscriber Identity Module (SIM) card (e.g., a machine-to-machine SIM card, a Universal Integrated Circuit Card, without limitation).

The power source 126 is configured to supply power to the delivery system 116 and the various electronic components, such as the one or more processors 118, memory 120, communication equipment 124, and the like. Power source 126 may be, as a non-limiting example, a power storage device (e.g., a battery, without limitation), a power inlet, a power regulator, or combination thereof.

In various embodiments, the automated medicament delivery device 114 may include one or more of: a sensor 128; an input device 130; and an output device 132. One or more sensors 128 may include one or more sensors chosen from among an accelerometer, a gyroscope, and an inertial measurement units (IMU) or similar sensors. One or more sensors 128 may further include one or more data sensors. Such data sensors may include one or more sensors chosen from a geographic sensor (e.g., a sensor for a satellite navigation system (e.g., the Global Positioning System (GPS) operated by the United States Department of Defense), without limitation), an Electrocardiogram sensor, a heart rate sensor, a skin conductance sensor, and the like. In one or more embodiments, one or more sensors 128 or automated medicament delivery device 114 more generally, may be configured to relate to one or more of a geographic location of the automated medicament delivery device 114, data regarding operation of the automated medicament delivery device 114, and patient data (e.g., electrical activity of the heart, heart rate, skin conductance, without limitation). One or more sensors 128 may include physical attribute sensors such as weight sensors, heart-rate sensors, temperature sensors, without limitation.

As described below, in various embodiments, the automated medicament delivery device 114 may be configured (e.g., via instructions of instructions 122 and one or more services 150) to utilize information provided by sensor 128 to determine automated medicament delivery device 114 is positioned on a user-body ("on-body sensor"), is operating correctly, or is operating in suitable environment, without limitation.

In one or more embodiments, the automated medicament delivery device 114 may be configured to monitor movement of a user-body at least partially based on information provided by sensors 128. In one or more embodiments, automated medicament delivery device 114 may be configured to determine that a user/patient of automated medicament delivery device 114 falls down at least partially based on information provided by sensor 128. In or more embodiments, automated medicament delivery device 114 may be configured to determine the amount of movement of a user-body, e.g., count steps, count distance traveled, without limitation.

In one or more embodiments, monitoring by automated medicament delivery device 114 may be modified based at least in part on a location on a user-body that the automated medicament delivery device 114 is positioned (an "on-body location"). In one or more embodiments, an on-body location may be received by automated medicament delivery device 114 via input device 130. In one or more embodiments, automated medicament delivery device 114 may prompt a patient via output device 132 to input an on-body location of the automated medicament delivery device 114. In some embodiments, the prompt may be provided at the user interface 148 of the handheld electronic computing device 138 (discussed below). In one or more embodiments, the one or more processors 118 may be configured (e.g., by instructions of instructions 122, without limitation) to determine an on-body location at least partially based on information provided by sensor 128 (e.g., based on a type of movement or a movement pattern respectively indicative of an on-body location, without limitation), user input, or a combination thereof.

The input device 130 may include a button (e.g., a mechanical button, capacitive button, without limitation), a gesture-based interface, and/or a microphone configured to receive an input from the patient (e.g., a button press, a tap, and/or a sound, without limitation). A gesture-based interface is an interface for recognizing a gesture-based input method. A gesture-based input method typically involves a sequence of movement also referred to herein as a "movement pattern." Non-limiting examples of movement patterns that may be utilized in a gesture-based input method include taps, shakes, rotation, swipes, and pinches. A gesture-based interface may include a combination of sensors configured to detect movement (e.g., a capacitive sensor, an accelerometer, a gyroscope, a magnetometer, a flex sensor, a pressure sensor, without limitation), and software configured to recognize the motion pattern from the movement data generated by the sensors and optionally interpret the motion pattern as a specific command.

The output device 132 may include one or more of: a display, an audio speaker, a Light Emitting Diode (LED), and/or vibration motor (e.g., a vibration transducer, without limitation) configured to provide a signal to the patient (e.g., a sound, a visual signal, such as light, and/or vibration, without limitation). The audio speaker may be configured to play multiple tones with various patterns. The one or more processors 118 may utilize a message parser to determine the message type, refer processes associated with medicament delivery to a main process or a main processor of the one or more processors 118, and refer special alert messages on to a message generator (e.g., a tone and pattern generator which drives the audio speaker or a vibration generator which drives the vibration motor, without limitation).

In various embodiments, the communication equipment 124 of the automated medicament delivery device 114 and communication equipment 112 of analyte sensor 102 may be configured to communicate with one or more services 150 via network 152. In various embodiments, the one or more services 150 may be provided by one or more computer servers or cloud computer servers. A respective server may be a dedicated secure endpoint configured to communicate with automated medicament delivery device 114 or analyte sensor 102. In various embodiments, the communication equipment 124 of the automated medicament delivery device 114 and communication equipment 112 of analyte sensor 102 may be configured to communicate via a short-range wireless protocol, and in response to the inability to communicate via the short-range wireless protocol, communicate via the one or more services 150 (e.g., the automated medicament delivery device 114 utilizes a IOT SIM card to communicate with the one or more services 150 to send and receive messages to and from the handheld electronic computing devices 138).

In various embodiments, the handheld electronic computing device 138 is configured to communicate with the automated medicament delivery device 114 and the analyte sensor 102. The handheld electronic computing device 138 may be chosen from among a dedicated electronic device, a smart phone, a tablet computer, a wearable device (e.g., a smart watch, without limitation), a cloud computing device, and the like.

The handheld electronic computing device 138 may include one or more processors 140, memory 142 that stores instructions 144 to be executed by the one or more processors 140, communication equipment 146, and a user interface 148. The one or more processors 140 and memory 142 may be configured/programmed to perform any of the operations discussed above, as well as other control operations for managing the automated medicament delivery device 114 and the analyte sensor 102.

The communication equipment 146 is configured to facilitate communication (e.g., wireless communication, without limitation) of the handheld electronic computing devices 138 with other devices, such as the automated medicament delivery device 114, the analyte sensor 102, and network 152. The communication may be wired or wireless communication, such as via a wireless networking protocol (e.g., Wi-Fi®, without limitation), a short-range wireless protocol (e.g., BLUETOOTH®, without limitation), a near-field communication standard, a cellular standard, or any other wireless optical or radio-frequency protocol. In some of these embodiments, the automated medicament delivery device 114 and the handheld electronic computing devices 138 are paired via the short-range wireless protocol (e.g., paired via BLUETOOTH®, without limitation) and successful message transmissions between the automated medicament delivery device 114 and the handheld electronic computing devices 138 may be acknowledged.

In various embodiments, the communication equipment 124 of the automated medicament delivery device 114 and the communication equipment 146 of the handheld electronic computing devices 138 are configured to communicate with and via one or more services 150. The one or more services 150 may be a dedicated secure endpoint configured to communicate with the handheld electronic computing devices 138 and other similar devices. In some of these various embodiments, the communication equipment 124 of the automated medicament delivery device 114 and the communication equipment 146 of the handheld electronic computing devices 138 are configured to communicate via the short-range wireless protocol, and in response to the inability to communicate via the short-range wireless protocol, communicate via the one or more services 150 (e.g., the automated medicament delivery device 114 utilizes a IOT SIM card to communicate with the one or more services 150 to send and receive messages to and from the handheld electronic computing devices 138).

The user interface 148 is configured to provide a user with information and obtain information from the user via one or more of a display, an audio speaker, an LED, a vibration motor, a button (e.g., a mechanical button, capacitive button, without limitation), a gesture-based interface, and the like.

In various embodiments, the one or more services 150 is configured to compile the alarms and data associated therewith (e.g., type of alarm, timestamp of the alarm, and geographic coordinates associated with the alarm, without limitation), analyze the data for common occurrences of alarms (e.g., particular locations where similar alarms occur, without limitation), and provide warnings to users (e.g., patients and caregivers, without limitation) about the common occurrences including the relevant information for the common occurrences (e.g., time window and location of the common occurrences, without limitation).

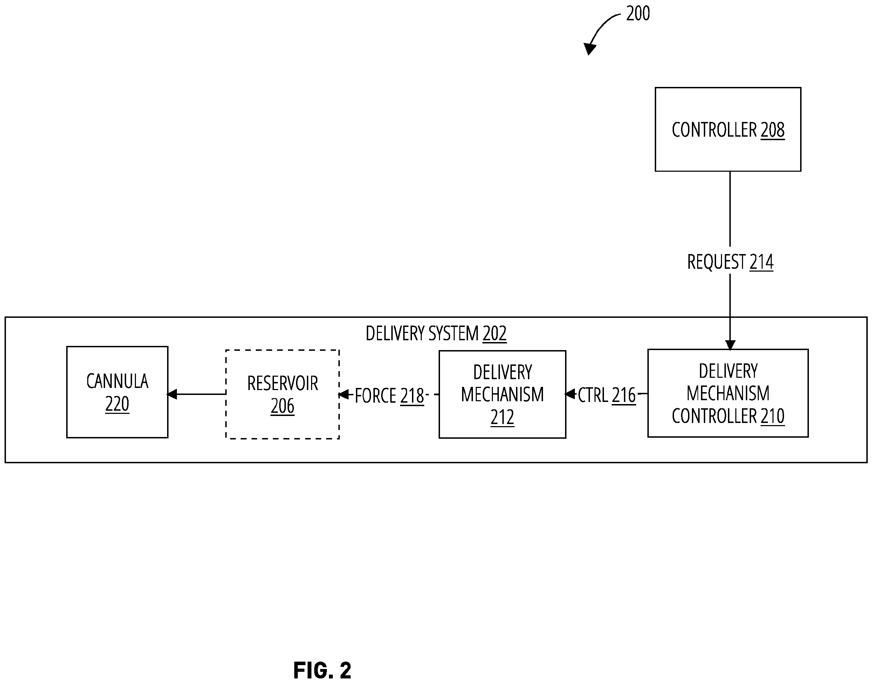

FIG. 2 is a block diagram of a medicament delivery system 200 for controlled administration of medicament to a user-body, in accordance with one or more examples.

The controller 208 is configured to manage automated medicament delivery device 114 and, more generally, administration of medicament to a user-body. In one or more embodiments, controller 208 may be implemented by instructions 122 and one or more processors 118 of automated medicament delivery device 114 of FIG. 1.

In various embodiments, controller 208 and delivery system 202 may be realized in different devices (e.g., controller 208 may be realized in a physically different device (or devices) than delivery system 202 is realized, without limitation), or in the same device. When realized in different devices, functionality of controller 208 and delivery system 202 may be implemented, at least in part, by respective memory and one or more processors of their respective devices. When realized in a same device, functionality of controller 208 and delivery system 202 may be implemented, at least in part, by like memory and like one or more processors, respective memory and respective one or more processors, or a combination thereof. Non-limiting examples of devices in which controller 208, or a portion thereof, may be realized include: a handheld electronic computing device, such as a dedicated electronic device, a smart phone, a tablet computer, a wearable device (e.g., a smart watch, without limitation), a cloud computing device, and the like.

In various embodiments, the controller 208 may be configured to receive analyte data (e.g., from analyte sensor 102, from one or more services 150, or both, without limitation) including analyte values. In one or more embodiments, controller 208 may determine information about analytes within a user-body at least partially based on analyte data, for example, amounts, trends, distributions, without limitation. The controller 208 may analyze information about analytes in a user-body and may present the information and/or analysis to a patient, caregiver, or healthcare provider, as a non-limiting example, via output device 132 of FIG. 1 or an application (e.g., executing on a personal computer, smart phone, cloud server, or combinations thereof).

In various embodiments, the controller 208 may be configured to receive information from sensors, inputs from the patient or a caregiver (e.g., when the patient ate a meal or when the patient exercised, without limitation), and inputs from other electronic devices (e.g., information from a smartwatch, without limitation) and to utilize such information as discussed herein. For example, in various embodiments, controller 208 may utilize some or a totality of such information to determine amounts of medicament to administer and timing of administration of medicament. Further, controller 208 may also be configured to determine requests, including request to administer dose 214, and send those requests to the automated medicament delivery device 114.

In various embodiments, controller 208 may be configured to determine a target dose amount to administer to a user of medicament delivery system 200. Controller 208 may determine a target dose amount at least partially based on therapy parameters, meal information, analyte values, and a control algorithm, without limitation.

In the context of insulin therapy to treat diabetes, therapy parameters may include insulin sensitivity factor (ISF), carbohydrate ratio (CR), amount of daily dose of long-acting insulin (LAI), a current glucose value, and derivatives thereof without limitation. The timing and target dose amounts associated with requests generated by controller 208 may be governed by one or more control algorithms, discussed below.

Controller 208 may send a request to administer dose to delivery system 202, and more specifically, delivery mechanism controller 210. The request to administer dose may include the target dose amount determined by controller 208.

The cannula 220 is insertable into a user-body (e.g., with a tip thereof positioned subcutaneously, without limitation) and is configured to provide medicament to a user-body (e.g., subcutaneously into the user-body, without limitation).

The reservoir 206 is configured to store and retain a medicament therein. As a non-limiting example, the reservoir 206 may be a hollow body, a chamber, a vial, without limitation. In various embodiments, reservoir 206 is a fluid reservoir for holding medicament and may be, as a non-limiting example, formed from the walls of a cartridge. In the cartridge example, delivery system 202 may include a chamber (i.e., a space or region defined within delivery system 202) configured to receive and hold a prefilled (prefilled with medicament) cartridge, eject an exhausted cartridge, and optionally receive a prefilled cartridge to replace (i.e., a replacement cartridge) the exhausted cartridge. Generally speaking, a volume of fluid in reservoir 206 will be greater in a pre-filled state than the volume in an exhausted state. Additionally or alternatively to the cartridge example, delivery system 202 is a multi-part delivery device where one of the two parts includes the reservoir 206 and the other one of the two parts includes the delivery mechanism controller 210. The other one of the two parts may optionally further include controller 208. Either one of the two parts may optionally include delivery mechanism 212 (e.g., a pump mechanism, without limitation). The one of the two parts that includes reservoir 206 is disposable (i.e., a "disposable part") and configured to be removable secured to the other part of medicament delivery system 200. When reservoir 206 is exhausted, the disposable part may be removed and a replacement part including a reservoir 206 optionally in a pre-filled state.

Delivery mechanism 212 is configured to urge fluid in reservoir 206 toward an interface for dispensing fluid (interface not shown). In various embodiments, delivery mechanism 212 may be positioned adjacent to reservoir 206. The delivery mechanism 212 is configured to cause an amount of the medicament to be administered to the user-body by causing the amount to flow from the reservoir 206 toward and into a user-body via cannula 220, which is in fluidic communication with the reservoir 206. In various embodiments, delivery mechanism 212 may utilize any suitable mechanism to generate positive displacement or negative displacement to transfer amounts of medicament from reservoir 206 toward cannula 220 and a user-body. Non-limiting examples of mechanisms include a ratchet gear pump, peristaltic pump, linear peristaltic pump, piston pump, gear pump, bellows pump, or diaphragm pump.

For example, delivery mechanism 212 may apply a force to an urging mechanism (e.g., a plunger, flexible-walled tube, without limitation) free to move within reservoir 206, and via such a force, move the urging mechanism in a direction that urges fluid in reservoir 206 toward the aforementioned interface. In one or more examples, delivery mechanism 212 may include an electrical motor (e.g., an AC or DC motor) that produces a force to, directly or indirectly, move the urging mechanism to perform a delivery action. A delivery action dispenses at a predetermined rate (i.e., a predictable amount of fluid over a predictable duration of time). The delivery mechanism 212 may be capable of multiple rates of delivery, and in one or more examples, may be preconfigured to use a same rate of delivery all the time, or, in some cases, may be provided discretion to determine a rate of delivery consistent with a target dose amount included with a request.

Such an electric motor may be a current controlled electric motor, voltage controlled electric motor, pulse-width controlled electric motor, or combination or sub combination thereof. Such an electronic motor may be directly or indirectly digitally controlled. The control signal 216 may be determined and generated by delivery mechanism controller 210 to correspond to a delivery action. A control signal 216 may also be referred to herein as a "command 216" or an "instruction 216."

Delivery mechanism controller 210 may generate control signals 216 corresponding to one or more delivery actions at least partially based on a request to administer dose 214 received from controller 208. Control signal 216 may include first control signals to cause delivery mechanism 212 to generate resultant force 218, and a second, different control signals to cause drive delivery mechanism 212 to not or stop generating force 218. Utilizing control signals 216, delivery mechanism controller 210 may control a length of a duration of time that delivery mechanism 212 produces force 218 and applies it to dispense fluid from reservoir 206, and indirectly, an amount of fluid dispensed from reservoir 206.

When delivery mechanism controller 210 generates control signal 216 in response to a request to administer dose 214 from controller 208, it may generate the control signal 216 at least partially based on a value of a target dose amount included with, or indicated by, request to administer dose 214. One or more delivery actions may be utilized to dispense an amount fluid corresponding to a dose amount determined by controller 208. For example, a fluid amount dispensed according to a delivery action may be less than a dose amount. Generally speaking, the delivery mechanism 212, and delivery system 202, are agnostic to the purpose for which fluid is dispensed and unaware of what constitutes a working amount of fluid to administer a dose, or series of doses, of medicament. So, while it may be desirable that a fluid amount dispensed according to one or more delivery actions will be exactly the same as a target dose amount, some negligible difference is specifically contemplated, and what is considered "negligible" will depend on specific operation conditions.

In one or more examples, delivery mechanism controller 210 may be configured to determine and generate feedback information about delivery actions, such as times of delivery actions and dispensed amounts, without limitation. Feedback information may be generated based on information generated by delivery mechanism 212 or by sensors utilized by delivery mechanism controller 210 to monitor operation of delivery mechanism 212 (sensors not depicted). For example, sensors to monitor mechanical movement, current consumption, a voltage profile of an electric motor, without limitation. Such information may be logged and provided to and stored at controller 208, handheld electronic computing device 138, or one or more services 150, without limitation, for e.g., later processing or reading, without limitation. For example, the logs can be processed to determine patterns that may be utilized to determine whether delivery system 202 is operating as expected (e.g., in a predictable manner, without limitation), and if a difference between actual and expected operation exceeds a threshold, delivery mechanism controller 210 may be updated (e.g., firmware, parameters, or both, of delivery mechanism controller 210 may be updated, without limitation) to compensate or correct for the difference. Additionally or alternatively to updating the firmware or parameters, in a multi-part system, one or more parts including delivery mechanism controller 210 or delivery mechanism controller 210 may be indicated as needing replacement (e.g., an alarm or alert is generated at delivery system 202, medicament delivery system 200, a mobile device or computer in communication therewith, without limitation).

Control Algorithm Architecture

Values of target dose amounts and timing of requests to administer the same generated by controller 208 may be governed by one or more control algorithms implemented at controller 208. Generally speaking, such a control algorithm may, via one or more control actions, try to cause an amount of analyte in the body (represented by values captured by, or at least partially based on, an analyte sensor or monitor, without limitation) to track a target amount of analyte (in control terms, the target amount of analyte is the "set point") in the body. The control actions may include amount and timing of administration of doses of medicament that functions as a therapeutic agent in the body.

In one or more examples, a control algorithm may employ a modular design in which core functionality may be separated from dependent functionality. Dependent functionality includes, as non-limiting examples, functionality that may be implementation-specific to a current environment, such as software abstraction for an analyte sensor. Such dependent functionality may include software services which interface with implementation-specific features that affect inputs or outputs to the control algorithm. Dependent functionality may include, as a non-limiting example, functionality for managing algorithm initialization and upload of administration history, managing the control algorithm's state and data variables, and maintaining cycle-to-cycle data utilized by the algorithm such as analyte values, current or historical. Dependent functionality may include functionality responsible for sending requests to administer doses to delivery system 202 which are determined by the control algorithm.

Transmission of data, including without limitation request to administer dose 214, may occur over wired, wireless, or a combination thereof communication paths, in a synchronous or asynchronous manner. In one or more examples, the control algorithm may include one or more layers to provide safety or other operational constraints (e.g., for edge case handling, without limitation).

In one or more examples, a control algorithm may determine a target dose amount that it includes with a request at least partially based on a dynamic model of the body's response, in terms of amount of analyte in the body, to administration of analyte to the body. The control algorithm may determine future amount of analyte or a change in amount of analyte over a predetermined duration of time for a respective dose amount and compare the determined future amount or change to a target amount or change. The algorithm may determine target dose amounts according to control intervals that occur according to a predetermined schedule, on-demand, or both. In one or more examples, control intervals may correspond to diurnal intervals such as day-night, weeks, days, twenty-four (24) hours, single hours, and sub-intervals of the same.

In some cases, the control algorithm may be or include a control algorithm that handles constraints, such as a model-predictive-control (MPC) algorithm. Non-limiting examples of constraints include: upper and lower bounds on analyte levels can be set to prevent dangerous hypo- or hyperglycemia; medicament delivery rates capable by delivery system 202 can be constrained to prevent over- or under-dosing; and considerations related to medicament-on-board to, e.g., prevent stacking of medicament doses waiting to work on analyte in the body (e.g., stacking of insulin waiting to work on glucose, without limitation).

One or more examples discussed herein may refer to administering medicament or a medicament therapy to a user or the user-body. Such discussion is intended to encompass examples where medicament or a medicament therapy is administered to a user by automated medicament delivery devices discussed herein, examples where requests to administer doses in accordance with administering medicament or medicament therapy to the user or user-body are generated by a controller and sent to a delivery device, and examples where instructions (e.g., control signals, without limitation) in accordance with dose amounts and timing included with such requests to administer doses are generated by a delivery mechanism controller and sent to a delivery mechanism.

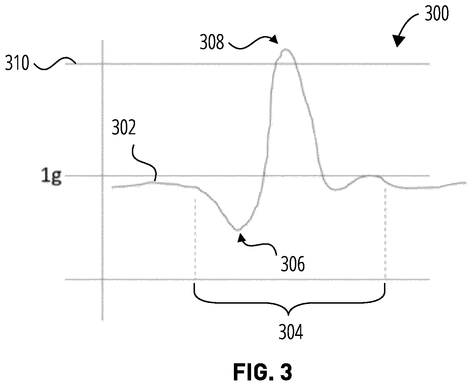

FIG. 3 is an example graph 300 of acceleration over time determined from motion data obtained by the sensor 128 of the automated medicament delivery device 114. Changes in resultant force 302 or other motion values, such as angular velocity, over time (e.g., within a time window 304, without limitation) may be indicative of a fall of a patient wearing the automated medicament delivery device 114. As illustrated in FIG. 3, under steady state, the resultant force 302 will be the force of gravity (1g). As a fall of a patient occurs, the resultant force 302 drops to a decreased resultant force 306 below the resultant force 302 caused by gravity, which is detectable in the motion data. For example, the resultant force 302 may drop to approximately half of the force of gravity while the patient wearing the automated medicament delivery device 114 is falling. As a result of impact by the patient with the floor or other objects the resultant force 302 elevates to an increased resultant force 308 above an acceleration threshold 310 indicative of a fall. For example, the acceleration threshold 310 may be double the force of gravity and an increased resultant force 308 above double the force of gravity may be indicative of a fall. However, merely detecting decreases and increases in motion values obtained from the sensor 128 may result in false positives. Various methods may be used to decrease the risk of a false positive in fall detection.

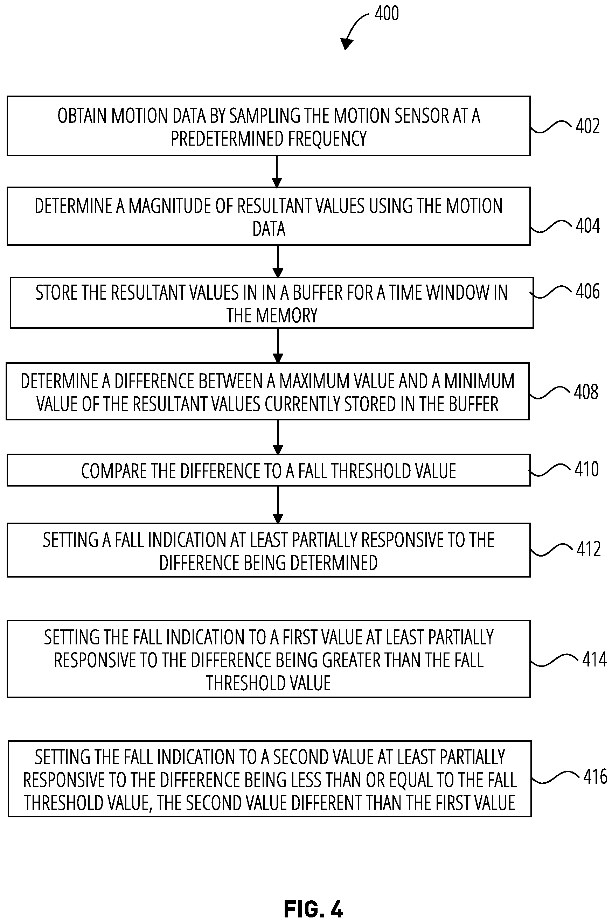

FIG. 4 is a flowchart of a method 400 for detecting a fall of a patient. The method 400 includes obtaining motion data by sampling a motion sensor of an automated medicament delivery device at a predetermined frequency at act 402. The automated medicament delivery device may be any automated medicament delivery device including embodiments of the automated medicament delivery device 114 disclosed herein. As discussed above, the motion sensor may be an accelerometer, a gyroscope, an inertial measurement unit (IMU), or similar sensors. In various embodiments, the motion data includes acceleration data, angular velocity data, or a combination thereof (e.g., data obtained from the motion sensor related to force, acceleration and/or the angular velocity). The sampling may be performed at any frequency (e.g., about 60 Hz, without limitation).

The method 400 also includes determining a magnitude of resultant values using the motion data at act 404. The resultant values may be acceleration, force, and/or angular velocity values derived from the motion data for each sample of the motion data taken. In various embodiments, the resultant values are determined by combining the magnitude of the resultant value in each direction of a coordinate system, such as the X, Y, and Z directions of the Cartesian coordinate system by using the equation: $V = \sqrt{V_x{}^2 + V_y{}^2 + V_z{}^2}$, where V is the resultant value.

The method 400 further includes storing the resultant values for a time window in the memory (e.g., in a buffer, in a register, without limitation) at act 406. The time window is a short time interval (e.g., a value chosen from within the range of about 0.5 seconds to about 1.5 seconds, such as 0.5 second, 1 second, 1.5 seconds, and the like, without limitation).

The method 400 yet further includes determining a difference between a maximum value and a minimum value of the resultant values currently stored in the buffer at act 408. The method 400 still further includes comparing the difference to a fall threshold value at act 410. The fall threshold value is a value indicative of a fall (e.g., the acceleration of gravity, the force of gravity, 100 degrees per second or factors thereof, such as at least 2 times any of the acceleration of gravity, the force of gravity, 100 degrees per second, without limitation). The fall threshold value may be predetermined at least partially based, as a non-limiting example, on specific operating conditions.

The method 400 also includes setting a fall indication at least partially responsive to the difference being determined at act 412. The method 400 further includes setting the fall indication to a first value at least partially responsive to the difference being greater than the fall threshold value at act 414. The method 400 yet further includes setting the fall indication to a second value at least partially responsive to the difference being less than or equal to the fall threshold value, the second value different than the first value at act 416.

In various embodiments, acts 412-416 include sending a signal to the handheld electronic computing device 138 that the fall indication has been set, and in particular, indicating that the fall indication has been set to the first value. In some of these various embodiments, the signal includes data related to the fall indication being set to the first value, such as the motion data, the resultant values, the maximum value, the minimum value, and the difference. The data related to the fall indication being set to the first value may be used to assess a potential severity of the fall (e.g., a larger difference is indicative of an increased impact which can potentially cause more severe injuries). The signal to the handheld electronic computing device 138 is sent via the communication equipment 124 via one or more of a wireless networking protocol (e.g., Wi-Fi®, without limitation), a short-range wireless protocol (e.g., BLUETOOTH®, without limitation), a near-field communication standard, a cellular standard, or any other wireless optical or radio-frequency protocol.

Some or a totality of the acts of method 400 may be performed by one or more of: the automated medicament delivery device 114, the handheld electronic computing device 138, or the one or more services 150. By way of non-limiting example of partitioning of acts of method 400, act 402 may be performed by the automated medicament delivery device 114 while acts act 404 to act 412 may be performed by the handheld electronic computing devices 138 or the one or more services 150.

In various embodiments, the method includes sending an alert to at least one party chosen from among a caregiver and an emergency service that a fall has been detected responsive to the fall indication being set to the first value. The handheld electronic computing device 138 or one or more services 150 may alert the at least one party upon receiving the signal from the automated medicament delivery device 114 that a fall has been detected. In some of these various embodiments, the method includes appending geographic coordinates (e.g., geotagging, without limitation) indicating a location of the fall to the alert. Appending the geographic coordinates to the alert provides an approximate location of the patient wearing the automated medicament delivery device 114 to the party that receives the alert. The geographic coordinates may be location data obtained from a satellite navigation system (e.g., GPS, without limitation) sensor of the automated medicament delivery device 114 or of the handheld electronic computing device 138. In some of these various embodiments, the method includes appending a timestamp to the alert. The timestamp may be obtained from the automated medicament delivery device 114, the one or more services 150, or the handheld electronic computing device 138.

The handheld electronic computing device 138 or one or more services 150 may send an acknowledgement to the automated medicament delivery device 114 that a caregiver or emergency service provider has been notified of the fall. The method may include, in response to receiving the acknowledgement, causing the automated medicament delivery device 114 to issue a notification. The notification may be issued by the output device 132 may include one or more of vibration and audible sound. The vibration/audio pattern may differ depending on which party notified of the fall.

In various embodiments, a single threshold chosen from among an acceleration threshold, a force threshold, and an angular velocity threshold. In other embodiments, multiple fall threshold values are required for a determination that a fall has been detected. For example, the method acts are conducted in parallel for acceleration/force data obtained from an accelerometer and for angular velocity data obtained from a gyroscope. By requiring multiple thresholds, the method may avoid triggering alarms while a patient routinely removing an automated medicament delivery device 114 from the user-body. Attachment sensors may also be used to detect when the automated medicament delivery device 114 is removed, which may be utilized to prevent an alert from being issued.

In various embodiments, the method includes increasing the predetermined frequency (e.g., the sample rate, without limitation) in response to a determination of an increased fall risk. In some of these various embodiments, an increased fall risk is determined by events including one or more of a missed medicament delivery, a medical condition determined from the data related to the one or more analytes within the user-body (e.g., hypoglycemia, without limitation), and the like. In some of these embodiments, the fall threshold value may be modified (e.g., lowered, without limitation) in response to the determination of an increased fall risk.

In various embodiments, the method includes setting the fall threshold value based in part on a location of the automated medicament delivery device 114 on the user-body. For example, the fall threshold value is set higher for the automated medicament delivery device 114 secured to the arm or shoulder the user-body than for the automated medicament delivery device 114 secured to the hip of the user-body.

In various embodiments, the method includes analyzing the components of the automated medicament delivery device 114 after the indication of the fall and, upon detecting damage to one or more components, issuing an alert indicating the damage. The alert may be output by the output device 132, by the handheld electronic computing device 138, or a combination thereof.

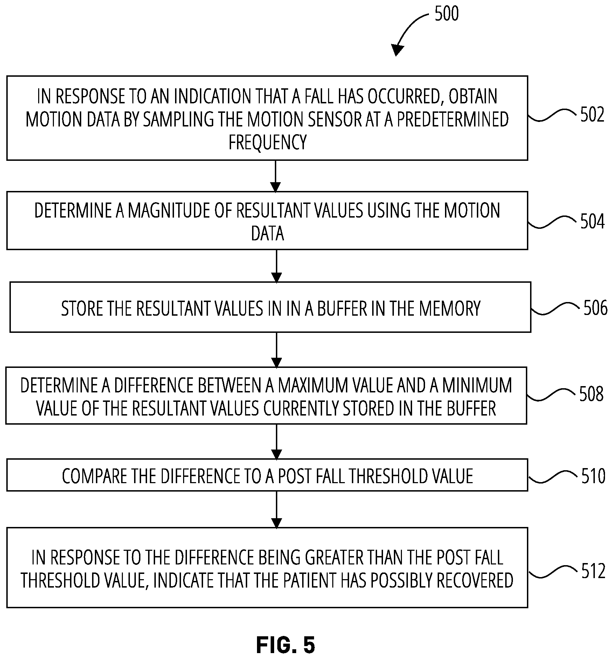

FIG. 5 is a flowchart of a method 500 for post fall analysis of a patient. The method 500 includes, in response to an indication that a fall has occurred, obtaining motion data by sampling the motion sensor at a predetermined frequency at act 502. The sampling performed in act 502 may be the same or similar to the sampling performed in act 402 of method 400. As discussed above, the motion sensor may be an accelerometer, a gyroscope, an IMU, or similar sensors. In various embodiments, the motion data includes acceleration data, angular velocity data, or a combination thereof (e.g., data obtained from the motion sensor related to force, acceleration and/or the angular velocity). The sampling may be performed at any frequency (e.g., 60 Hz, without limitation).

The method 500 also includes determining a magnitude of resultant values using the motion data. The determining performed in act 504 may be the same or similar to the determining performed in act 404 of method 400. As described with regards to act 404, the resultant values may be acceleration, force, and/or angular velocity values derived from the motion data for each sample of the motion data taken. In various embodiments, the resultant values are determined by combining the magnitude of the resultant value in each of the X, Y, and Z directions of the Cartesian coordinate system or any similar coordinate system, such as by using the equation:

$$V = \sqrt{V_x^2 + V_y^2 + V_z^2}$$

, where V is the resultant value.

The method 500 further includes storing the resultant values in a buffer in the memory at act 506. In various embodiments, the buffer in method 500 may be configured to store the resultant values for the time window of method 400 or for a longer time period than the time window of method 400.

The method 500 yet further includes determining a difference between a maximum value and a minimum value of the resultant values currently stored in the buffer at act 508. The method still further includes comparing the difference to a post fall threshold value at act 510. The post fall threshold value is a value greater than zero (as a value of about zero would be indicative of a lack of recovery or movement of the patient) and less than the fall threshold value of method 400. The post fall threshold value is a value indicative of a recovery from a fall (e.g., a value greater than zero and one of: below the acceleration of gravity and below the force of gravity, 0.2g, or 50 degrees per second, without limitation).

The method 500 also includes, in response to the difference being greater than the post fall threshold value, indicating that the patient has possibly recovered at act 512. In various embodiments, act 512 includes sending a signal to the handheld electronic computing device 138 that a possible recovery has been detected.

In various embodiments, a single threshold chosen from among an acceleration threshold, a force threshold, and an angular velocity threshold. In other embodiments, multiple post fall threshold values are required for a determination that a possible recovery has been detected. For example, the method acts are conducted in parallel for acceleration/force data obtained from an accelerometer and for angular velocity data obtained from a gyroscope.

The method 500 may be performed by the automated medicament delivery device 114 or may be performed by a combination of the automated medicament delivery device 114 with one or more of the one or more services 150 and the handheld electronic computing device 138. For example, act 502 may be performed by the automated medicament delivery device 114 while acts 404-412 may be performed by the one or more services 150, the handheld electronic computing device 138, or a combination thereof.

The method 500 may include receiving an input from the patient via an input device that the patient has recovered from the detected fall.

The method 500 may also include determining a pose of the patient after the determination that the fall occurred and utilizing the pose to determine whether the patient has recovered from the fall. If the patient is upright (e.g., standing, without limitation), recovery may be suggested, while a patient that is prone (e.g., laying down, without limitation).

In various embodiments, the method 400 and the method 500 are performed on a dedicated processor of the one or more processors 118, while medicament administration is controlled by a primary processor of the one or more processors 118.

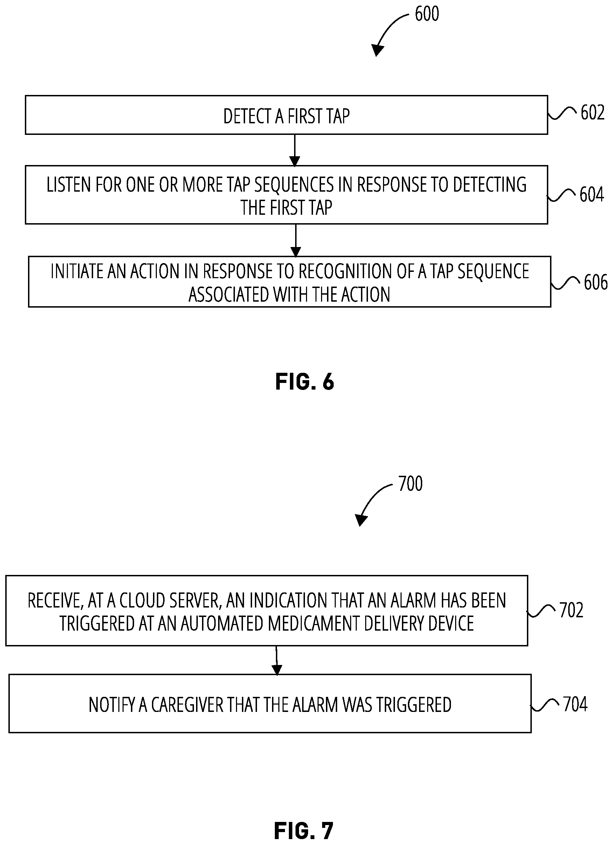

FIG. 6 is a flowchart of a method 600 for receiving commands at an automated medicament delivery device. The method 600 includes detecting a first tap at act 602. In various embodiments, the method includes maintaining the input device (e.g., an accelerometer, without limitation)in a low power mode until detection of the first tap. In the low power mode, low power/standby processing will perform simple processing (e.g., monitor for a threshold force/acceleration, without limitation). By maintaining the input device in a low power mode while listening for the first tap, power savings can be attained, ensuring the battery life of the automated medicament delivery device lasts as long as expected.

The method 600 also includes listening for one or more tap sequences in response to detecting the first tap at act 604. The tap sequence may be a single tap, multiple taps, a combination of short and long taps, and the like.

In various embodiments, the method includes switching the input device to a high-power mode in response to act 602 (e.g., an accelerometer is woken up to the high-power mode upon detection of the first tap, without limitation). In the high-power mode, high power/active sensor processing is performed at a high sampling rate. The high-power mode includes a higher sampling rate than the low power mode, which may improve accuracy of processing taps applied to the automated medicament delivery device. In some of these various embodiments, the method includes maintaining the input device in the high-power mode for a predetermined time, and in response to no subsequent tap being detected, switching the input device back to the low power mode. In various embodiments, separate processors are used for each mode. In particular, the low power mode is performed by a low power processor with a high-power processor in a sleep mode. Switching to the high-power mode includes waking up the high-power processor. The high-power mode is then performed by the high-power processor.

The method 600 further includes initiating an action in response to recognition of a tap sequence associated with the action at act 606. In various embodiments, act 606 includes sending a signal to the handheld electronic computing device to initiate the action. Various actions may be initiated depending on the tap sequence recognized. Each action is associated with a unique tap sequence. In various embodiments, each of the one or more tap sequences is associated with a distinct action with at least one action chosen from among causing the handheld electronic computing device to emit a notification, change a setting on the handheld electronic computing device, silence a notification on the automated medicament delivery device, switch of an alert on the automated medicament delivery device, snooze an alert on the automated medicament delivery device, silence a notification on the handheld electronic computing device, issue an alarm (e.g., indicating that the patient is in distress), provide a response to a notification (e.g., yes or no response based on the tap sequence, without limitation), and the like. The notification may include emitting a sound and/or vibration, which may help a patient find a misplaced handheld electronic computing device. The setting may include a sound setting of the handheld electronic computing device (e.g., changing the phone to the silent mode, the sound mode, or a vibration mode, without limitation). The notifications may include alerts related to a health condition of the patient and alerts associated with other functionalities of the handheld electronic computing device (e.g., phone call or text message notification, without limitation). The alert may be active for a predetermined timeframe (e.g., 15, 30, 45, or 60 seconds, without limitation).

FIG. 7 is a flowchart of a method 700 for providing an alert. The method 700 includes receiving, at a cloud server, an indication that an alarm has been triggered at an automated medicament delivery device at act 702. The indication may be sent directly from the automated medicament delivery device (e.g., via an IOT SIM included in the communication equipment of the automated medicament delivery device, without limitation) or may be sent by the handheld electronic computing device , such as using an embodiment of the method 800 as outlined below.

One or more alarms may be triggered at the automated medicament delivery device or the handheld electronic computing device . In various embodiments, the one or more alarms is chosen from among an indication of a fall of the patient (refer to the method 400), an indication of a recovery of the patient (refer to the method 500), an alarm associated with a health condition of the patient (e.g., hypoglycemia or a missed medicament delivery, without limitation), an alarm triggered by the patient (e.g., via the method 600, without limitation), an alarm associated with a condition of the automated medicament delivery device (e.g., reservoir for containing medicament is empty, automated medicament delivery device is damaged, electro-static discharge, without limitation), and the like.

In various embodiments, the alarm includes geographic coordinates (e.g., geotagging, without limitation) appended thereto indicating a location where the alarm occurred. The geographic coordinates may be GPS data obtained from a GPS sensor of the handheld electronic computing device or the automated medicament delivery device.

The method 700 also includes notifying a caregiver that the alarm was triggered at act 704. In various embodiments, notifying the caregiver includes sending the notification to an electronic device of the caregiver (e.g., a supplemental handheld electronic computing device , a mobile input device, and/or a computer, without limitation) from the cloud server. In some of these various embodiments, the notification is sent via at least one notification method chosen from among a push notification sent to an application operating on the electronic device of the caregiver, a message sent over a cellular network (e.g., a Short Message Service (SMS) message, without limitation), a message sent via the internet (e.g., an internet based messaging service, without limitation), a phone call, a voice message, an email, and an Application Programming Interface (API) notification. The caregiver or caregivers may specify which of the notification methods the caregiver would like the cloud server to use to notify the caregiver that the alarm was triggered, which may be customizable for each caregiver receiving notifications for a particular patient.

In various embodiments, the method includes compiling the alarms received at the cloud server and data associated therewith (e.g., type of alarm, timestamp of the alarm, and geographic coordinates associated with the alarm, without limitation), analyzing the data for common occurrences of the alarms (e.g., particular locations where similar alarms occur, without limitation), and providing warnings to users (e.g., patients and caregivers, without limitation) about the common occurrences including the relevant information for the common occurrences (e.g., time window and location of the common occurrences, without limitation).

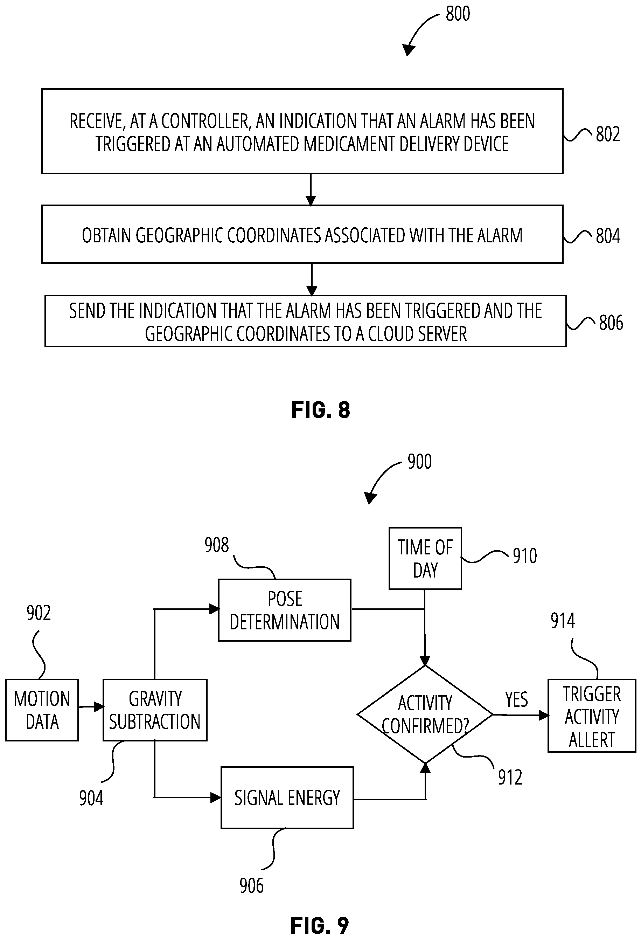

FIG. 8 is a flowchart of a method 800 for processing an alert. The method 800 includes receiving, at a handheld electronic computing device 138, an indication that an alarm has been triggered at an automated medicament delivery device at act 802. In various embodiments, the alarm is chosen from among an indication of a fall of the patient (refer to the method 400), an indication of a recovery of the patient (refer to the method 500), an alarm associated with a health condition of the patient (e.g., hypoglycemia or a missed medicament delivery, without limitation), an alarm triggered by the patient (e.g., via the method 600, without limitation), a condition of the automated medicament delivery device (e.g., reservoir for containing medicament is empty, automated medicament delivery device is damaged, electro-static discharge, without limitation), and the like.

The method 800 also includes obtaining geographic coordinates associated with the alarm at act 804. The geographic coordinates may be obtained from a GPS sensor of the handheld electronic computing device 138 or the automated medicament delivery device (e.g., querying the operating system of the handheld electronic computing device 138 to obtain the GPS data, without limitation) at a time the alarm is triggered or at a time the indication is received at the handheld electronic computing device 138.

The method 800 further includes sending the indication that the alarm has been triggered and the geographic coordinates to a cloud server at act 806. The handheld electronic computing device 138 may be configured to communicate with the cloud server over any combination of networks (e.g., Wi-Fi network, cellular network, internet, without limitation).

FIG. 9 is a flowchart of a process 900 for triggering an alert in response to an identified activity of a patient. In various embodiments, the activity is chosen from among inactivity of the patient, sleep, and exercise. The process 900 includes obtaining motion data by sampling a motion sensor of an automated medicament delivery device at act 902. The motion sensor may be an accelerometer, a gyroscope, an inertial measurement units (IMU), or similar sensors. In various embodiments, the motion data includes acceleration data, angular velocity data, or a combination thereof (e.g., data obtained from the motion sensor related to force, acceleration and/or the angular velocity). The sampling may be performed at any frequency (e.g., 60 Hz, without limitation).The motion data may be the same or similar to the motion data obtained in method 400 or method 500. The motion data may be sampled at the same or a similar frequency to the method 400 or method 500.

The process 900 also includes determining a magnitude of dynamic motion data by subtracting a value of gravity from the motion data at act 904 (e.g., subtracting the value of the force, acceleration, and/or the angular velocity caused by gravity from the dynamic motion data, without limitation). The process 900 further includes determining a signal energy from the magnitude of dynamic motion data at act 906. The signal energy may be the integral of the magnitude of the motion data. The measure of signal energy may be the integral of the square of the signal over a time window, and measures of signal energy may be computed over time windows of different lengths. In various embodiments, the process 900 also includes determining a pose of the patient at act 908 using the magnitude of the dynamic motion data at act 908. In some of these various embodiments, the motion data includes a gravity signal measured and provided by a time averaged accelerometer. The gravity signal points vertically downward irrespective of the orientation of the accelerometer. A projection of the gravity vector on the x,y,z axes of the accelerometer may provide an indication of the pose in which the device/user-body is oriented. In various embodiments, pose transitions are computed by examining changes in the orientation of the gravity vector (e.g., changes from vertical to supine or vertical to sitting, without limitation). The pose determination may be determined based on a position of the automated medicament delivery device. The gravity vector for a person sitting or laying down may be different for an automated medicament delivery device worn on the thigh of the user-body than the gravity vector for an automated medicament delivery device worn on an arm or torso of the user-body. For example, the signatures of the gravity vector when worn on the thigh of the user-body from standing to sitting to supine may be quite clear. However, the signatures of the gravity vector when worn on the forearm of the user-body standing to supine may be clear whereas standing to sitting may have the same or similar signatures. In various embodiments, the motion data also includes gyro angular rate angles that may be used in conjunction with the accelerometer to track poses. Based on an initial pose assumption, numerical integration may be carried out to determine pose. Periodic updates may minimize the error in pose determination. In various embodiments, 6 Degree of freedom Kalman filters are utilized for pose tracking. In these various embodiments, the process 900 further includes obtaining a time of day at act 910.

The process 900 includes determining whether an activity of the patient has occurred based at least in part by comparing the signal energy to an activity threshold value at act 912. In various embodiments, exercise is determined by comparing the signal energy to an exercise threshold value, and in response to the signal energy being above the exercise threshold value, indicating that the patient is exercising. In various embodiments, inactivity is determined by comparing the signal energy to an inactivity threshold value, determining that the signal energy has been below the inactivity threshold value for a predetermined time threshold, and in response to the signal energy being below the inactivity threshold value for the predetermined time threshold, the pose being different than a sleeping posture, and the time of day being daytime, indicating that the patient is inactive. In various embodiments, sleep is determined by comparing the signal energy to the inactivity threshold value, determining that the signal energy has been below the inactivity threshold value for a predetermined time threshold, and in response to the signal energy being below the inactivity threshold value for the predetermined time threshold, the pose being that of a sleeping posture, and the time of day being nighttime, indicating that the patient is sleeping.

The process 900 further includes triggering an activity alert in response to a determination that the activity of the patient has occurred at 914. The activity alert may be processed in any manner disclosed herein, such as sending the activity alert to the handheld electronic computing device 138.

The handheld electronic computing device 138 may be configured to perform various actions in response to receiving the activity alert. In various embodiments, in response to receiving the exercise activity alert, the handheld electronic computing device 138 is configured to perform at least one of set an activity mode in the control of medicament administration (e.g., hypoglycemia protection for insulin delivery, without limitation) and set an alarm for a reminder to the patient (e.g., reminding the patient to eat for further hypoglycemia protection).

Other sensors, such as an EKG sensor, heart rate sensor, blood pressure sensor, and the like may also be utilized to obtain data, which can be compared to thresholds to trigger various alerts and alarms.

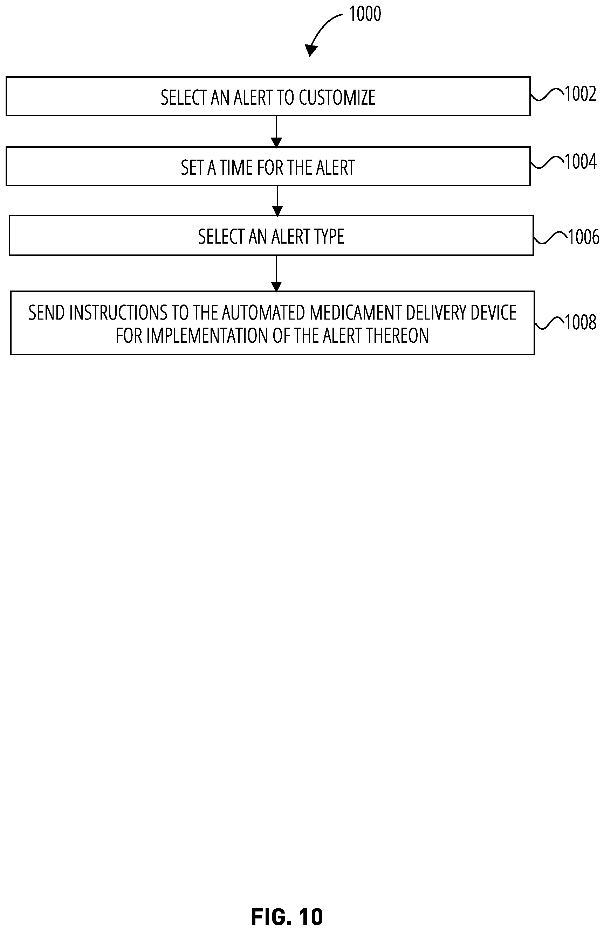

FIG. 10 is a flowchart of a method 1000 for customizing alerts provided at an automated medicament delivery device. The method 1000 may be performed on a user interface of the handheld electronic computing device 138.

The method 1000 includes selecting an alert to customize at act 1002. In various embodiments, the alert is selected from among a wake-up alarm, a meal alarm, a snack alarm, and any other alerts identified herein. The method 1000 also includes setting a time for the alert at act 1004. The time may include any of specific date, day, hour, and minutes.

The method 1000 further includes selecting an alert type at act 1006. The alert type may be selected from an audio pattern, a vibration pattern, a pattern with a combination of audio and vibration patterns, and a custom pattern. In various embodiments, the method includes receiving inputs defining a combination of alert outputs to define a custom pattern for the alert type, generating the custom pattern, and causing the custom pattern to output at the automated medicament delivery device. The custom pattern may be any combination of audio and vibration outputs.

The method 1000 still further includes the act 1008 of sending instructions to the automated medicament delivery device for implementation of the alert thereon at act 1008.

In the detailed description, reference is made to the accompanying drawings, which form a part hereof, and in which are shown, by way of illustration, specific examples of embodiments in which the present disclosure may be practiced. These embodiments are described in sufficient detail to enable a person of ordinary skill in the art to practice the present disclosure. However, other embodiments may be utilized, and structural, material, and process changes may be made without departing from the scope of the disclosure.

The illustrations presented herein are not meant to be actual views of any particular method, system, device, or structure, but are merely idealized representations that are employed to describe the embodiments of the present disclosure. The drawings presented herein are not necessarily drawn to scale. Similar structures or components in the various drawings may retain the same or similar numbering for the convenience of the reader; however, the similarity in numbering does not mean that the structures or components are necessarily identical in size, composition, configuration, or any other property.

The description may include examples to help enable one of ordinary skill in the art to practice the disclosed embodiments. The use of the terms "exemplary," "by example," and "for example," means that the related description is explanatory, and though the scope of the disclosure is intended to encompass the examples and legal equivalents, the use of such terms is not intended to limit the scope of an embodiment or this disclosure to the specified components, steps, features, functions, or the like.

It will be readily understood that the components of the embodiments as generally described herein and illustrated in the drawing could be arranged and designed in a wide variety of different configurations. Thus, the description of various embodiments is not intended to limit the scope of the present disclosure but is merely representative of various embodiments.

While the various aspects of the embodiments may be presented in drawings, the drawings are not necessarily drawn to scale unless specifically indicated.

Furthermore, specific implementations shown and described are only examples and should not be construed as the only way to implement the present disclosure unless specified otherwise herein. Elements, circuits, and functions may be shown in block diagram form in order not to obscure the present disclosure in unnecessary detail. Conversely, specific implementations shown and described are exemplary only and should not be construed as the only way to implement the present disclosure unless specified otherwise herein. Additionally, block definitions and partitioning of logic between various blocks is exemplary of a specific implementation. It will be readily apparent to one of ordinary skill in the art that the present disclosure may be practiced by numerous other partitioning solutions. For the most part, details concerning timing considerations and the like have been omitted where such details are not necessary to obtain a complete understanding of the present disclosure and are within the abilities of persons of ordinary skill in the relevant art.

In the Brief Summary and in the Detailed Description, the claims, and in the accompanying drawings, reference is made to particular features (including method acts) of the present disclosure. It is to be understood that the disclosure includes all possible combinations of such particular features. For example, where a particular feature is disclosed in the context of a particular embodiment, or a particular claim, that feature can also be used, to the extent possible, in combination with and/or in the context of other particular aspects and embodiments described herein.

Those of ordinary skill in the art would understand that information and signals may be represented using any of a variety of different technologies and techniques. Some drawings may illustrate signals as a single signal for clarity of presentation and description. It will be understood by a person of ordinary skill in the art that the signal may represent a bus of signals, wherein the bus may have a variety of bit widths and the present disclosure may be implemented on any number of data signals including a single data signal.

The various illustrative methods, logical blocks, modules, and circuits described in connection with the embodiments of the system 100, and in particular, the automated medicament delivery device 114 and the handheld electronic computing device 138, disclosed herein may be implemented or performed with a general purpose processor, a special purpose processor, a digital signal processor (DSP), an Integrated Circuit (IC), an Application Specific Integrated Circuit (ASIC), a Field Programmable Gate Array (FPGA) or other programmable logic device, discrete gate or transistor logic, discrete hardware components, or any combination thereof designed to perform the functions described herein. A general-purpose processor (may also be referred to herein as a host processor or simply a host) may be a microprocessor, but in the alternative, the processor may be any conventional processor, controller, microcontroller, or state machine. A processor may also be implemented as a combination of computing devices, such as a combination of a DSP and a microprocessor, a plurality of microprocessors, one or more microprocessors in conjunction with a DSP core, or any other such configuration. A general-purpose computer including a processor is considered a special-purpose computer while the general-purpose computer is configured to execute computing instructions (e.g., software code) related to embodiments of the present disclosure.

The embodiments may be described in terms of a process that is depicted as a flowchart, a flow diagram, a structure diagram, or a block diagram. Although a flowchart may describe operational acts as a sequential process, many of these acts can be performed in another sequence, in parallel, or substantially concurrently. In addition, the order of the acts may be rearranged. A process may correspond to a method, a thread, a function, a procedure, a subroutine, a subprogram, other structure, or combinations thereof. Furthermore, the methods disclosed herein may be implemented in hardware, software, or both. If implemented in software, the functions may be stored or transmitted as one or more instructions or code on computer-readable media. Computer-readable media includes both computer storage media and communication media including any medium that facilitates transfer of a computer program from one place to another.

As used herein, the term "substantially" in reference to a given parameter, property, or condition means and includes to a degree that one skilled in the art would understand that the given parameter, property, or condition is met with a small degree of variance, such as within acceptable manufacturing tolerances. For example, a parameter that is substantially met may be at least about 90% met, at least about 95% met, or even at least about 99% met.

As used herein, the term "about" used in reference to a given parameter is inclusive of the stated value and has the meaning dictated by the context (e.g., it includes the degree of error associated with measurement of the given parameter, as well as variations resulting from manufacturing tolerances, etc.).

As used herein, the terms "adapted," "configured," and "configuration" refers to a size, a shape, a material composition, a material distribution, orientation, and arrangement of at least one feature (e.g., one or more of at least one structure, at least one material, at least one region, at least one device) facilitating use of the at least one feature in a pre-determined way.

As used herein, the term "may" with respect to a material, structure, feature, function, or method act indicates that such is contemplated for use in implementation of an embodiment of the disclosure, and such term is used in preference to the more restrictive term "is" so as to avoid any implication that other compatible materials, structures, features, functions, and methods usable in combination therewith should or must be excluded.

Non-limiting illustrative embodiments of this disclosure may include:

Embodiment 1: A method, comprising: obtaining motion data by sampling a motion sensor of an automated medicament delivery device at a predetermined frequency; determining a magnitude of resultant values using the motion data; storing the resultant values in a buffer for a time window in a memory; determining a difference between a maximum value and a minimum value of the resultant values currently stored in the buffer; comparing the difference to a fall threshold value; and setting a fall indication at least partially responsive to the comparison.

Embodiment 2: The method according to Embodiment 1, wherein setting a fall indication at least partially responsive to the comparison comprises: setting the fall indication to a first value at least partially responsive to the difference being greater than the fall threshold value.

Embodiment 3: The method according to any of Embodiments 1 and 2, wherein setting a fall indication at least partially responsive to the comparison comprises: setting the fall indication to a second value at least partially responsive to the difference being less than or equal to the fall threshold value, the second value different than the first value.

Embodiment 4: The method according to any of Embodiments 1 through 3, wherein the motion sensor includes at least one sensor chosen from among an accelerometer, a gyroscope, and an inertial measurement unit, and wherein the motion data includes data chosen from among acceleration data, angular velocity data, and a combination of motion data and acceleration data.

Embodiment 5: The method according to any of Embodiments 1 through 4, wherein the resultant values are determined by combining the magnitude of the resultant value in each direction of a coordinate system.

Embodiment 6: The method according to any of Embodiments 1 through 5, wherein a timer interval is a short time interval with a value chosen from within a range of about 0.5 seconds to about 1.5 seconds.

Embodiment 7: The method according to any of Embodiments 1 through 6, wherein the fall threshold value is at least 2 times any of an acceleration of gravity, a force of gravity, and 100 degrees per second.

Embodiment 8: The method according to any of Embodiments 1 through 7, wherein indicating that the fall has been detected includes sending a signal to a handheld electronic computing device associated with the automated medicament delivery device that the fall has been detected.

Embodiment 9: The method according to any of Embodiments 1 through 8, wherein the signal includes data related to the fall including the motion data and the resultant values.

Embodiment 10: The method according to any of Embodiments 1 through 9, further comprising sending an alert to at least one party chosen from among a caregiver and an emergency service that a fall has been detected.

Embodiment 11: The method according to any of Embodiments 1 through 10, further comprising appending geographic coordinates indicating a location of the fall to the alert.

Embodiment 12: The method according to any of Embodiments 1 through 11, further comprising causing the automated medicament delivery device to issue a notification indicating that the at least one party received the alert.

Embodiment 13: The method according to any of Embodiments 1 through 12, further comprising increasing the predetermined frequency in response to a determination of an increased fall risk.

Embodiment 14: The method according to any of Embodiments 1 through 13, further comprising setting the fall threshold value based in part on a location of the automated medicament delivery device on a user-body.

Embodiment 15: The method according to any of Embodiments 1 through 14, further comprising analyzing components of the automated medicament delivery device after the indication of the fall and, upon detecting damage to one or more components, issuing an alert indicating the damage.

Embodiment 16: The method according to any of Embodiments 1 through 15, further comprising: in response to the indication that a fall has occurred, comparing the difference to a post fall threshold value; and in response to the difference being greater than the post fall threshold value, indicating that a patient has possibly recovered.

Embodiment 17: The method according to any of Embodiments 1 through 16, wherein the post fall threshold value is a value greater than zero and less than the fall threshold value.

Embodiment 18: The method according to any of Embodiments 1 through 17, further comprising: detecting a first tap at the motion sensor; listening for one or more tap sequences in response to detecting the first tap; and initiating, at the automated medicament delivery device, an action in response to recognition of a tap sequence associated with the action.

Embodiment 19: The method according to any of Embodiments 1 through 18, further comprising: receiving, at a cloud server, an indication that an alarm has been triggered at an automated medicament delivery device; and notifying a caregiver that the alarm was triggered.

Embodiment 20: The method according to any of Embodiments 1 through 19, wherein the alarm is the indicating that the fall has been detected, and wherein notifying the caregiver includes providing the caregiver with a geographic location of where the fall occurred.

Embodiment 21: An automated medicament delivery device, comprising: a motion sensor; one or more processors; and memory including instructions, when executed, cause the one or more processors to: obtain motion data by sampling the motion sensor at a predetermined frequency; determine a magnitude of resultant values using the motion data; store the resultant values in in a buffer for a time window in the memory; determine a difference between a maximum value and a minimum value of the resultant values currently stored in the buffer; compare the difference to a fall threshold value; and in response to the difference being greater than the fall threshold value, indicate that a fall has been detected.

Embodiment 22: The automated medicament delivery device according to Embodiment 21, wherein the motion sensor includes at least one sensor chosen from among an accelerometer, a gyroscope, and an inertial measurement unit, and wherein the motion data includes data chosen from among acceleration data, angular velocity data, and a combination of acceleration data and angular velocity data.

Embodiment 23: The automated medicament delivery device according to any of Embodiments 21 and 22, wherein the resultant values are determined by combining the magnitude of the resultant value in each direction of a coordinate system.

Embodiment 24: The automated medicament delivery device according to any of Embodiments 21 through 23, wherein a timer interval is a short time interval with a value chosen from within a range of about 0.5 seconds to about 1.5 seconds.

Embodiment 25: The automated medicament delivery device according to any of Embodiments 21 through 24, wherein the fall threshold value is at least 2 times any of an acceleration of gravity, a force of gravity, and 100 degrees per second.

Embodiment 26: The automated medicament delivery device according to any of Embodiments 21 through 25, wherein indicating that the fall has been detected includes sending a signal to a handheld electronic computing device associated with the automated medicament delivery device that the fall has been detected.

Embodiment 27: The automated medicament delivery device according to any of Embodiments 21 through 26, wherein the signal includes data related to the fall including the motion data and the resultant values.

Embodiment 28: The automated medicament delivery device according to any of Embodiments 21 through 27, further comprising at least one output device chosen from among an audio speaker and a vibration motor, and wherein the instructions, when executed, further cause the one or more processors to issue a notification indicating that at least one party chosen from among a caregiver and an emergency service received an alert that a fall has been detected.

Embodiment 29: The automated medicament delivery device according to any of Embodiments 21 through 28, wherein the instructions, when executed, further cause the one or more processors to increase the predetermined frequency in response to a determination of an increased fall risk.

Embodiment 30: The automated medicament delivery device according to any of Embodiments 21 through 29, wherein the instructions, when executed, further cause the one or more processors to set the fall threshold value based in part on a location of the automated medicament delivery device on a user-body.

Embodiment 31: The automated medicament delivery device according to any of Embodiments 21 through 30, wherein the instructions, when executed, further cause the one or more processors to: in response to the indication that a fall has occurred, compare the difference to a post fall threshold value; and in response to the difference being greater than the post fall threshold value, indicate that a patient has possibly recovered.

Embodiment 32: The automated medicament delivery device according to any of Embodiments 21 through 31, wherein the post fall threshold value is a value greater than zero and less than the fall threshold value.

Embodiment 33: The automated medicament delivery device according to any of Embodiments 21 through 32, wherein the instructions, when executed, further cause the one or more processors to: detect a first tap at the motion sensor; listen for one or more tap sequences in response to detecting the first tap; and initiate an action in response to recognition of a tap sequence associated with the action.

Embodiment 34: A method, comprising: detecting a first tap at a motion sensor of an automated medicament delivery device; listening for one or more tap sequences in response to detecting the first tap; and initiating, at the automated medicament delivery device, an action in response to recognition of a tap sequence associated with the action.

Embodiment 35: The method according to Embodiment 34, wherein each of the one or more tap sequences is associated with a distinct action associated with a unique tap sequence.

Embodiment 36: The method according to any of Embodiments 34 and 35, wherein at least one action is chosen from among causing a handheld electronic computing device associated with the automated medicament delivery device to emit a notification, change a setting on the handheld electronic computing device, silence a notification on the automated medicament delivery device, switch of an alert on the automated medicament delivery device, snooze an alert on the automated medicament delivery device, silence a notification on the handheld electronic computing device, issue an alarm, provide a response to a notification.

Embodiment 37: The method according to any of Embodiments 34 through 36, further comprising: maintaining an input device in a low power mode until detection of the first tap; and switching the input device to a high-power mode in response to detecting the first tap.

Embodiment 38: The method according to any of Embodiments 34 through 37, wherein the high-power mode includes a higher sampling rate than the low power mode.

Embodiment 39: The method according to any of Embodiments 34 through 38, further comprising: maintaining the input device in the high-power mode for a predetermined time; and in response to no subsequent tap being detected, switching the input device back to the low power mode.

Embodiment 40: The method according to any of Embodiments 34 through 39, wherein initiating the action includes sending a signal to a handheld electronic computing device associated with the automated medicament delivery device to initiate the action at the handheld electronic computing device.

Embodiment 41: An automated medicament delivery device, comprising: a motion sensor; one or more processors; and memory including instructions, when executed, cause the one or more processors to: detect a first tap at the motion sensor; listen for one or more tap sequences in response to detecting the first tap; and initiate an action in response to recognition of a tap sequence associated with the action.

Embodiment 42: The automated medicament delivery device according to Embodiment 41, wherein each of the one or more tap sequences is associated with a distinct action associated with a unique tap sequence.

Embodiment 43: The automated medicament delivery device according to any of Embodiments 41 and 42, wherein at least one action is chosen from among causing a handheld electronic computing device associated with the automated medicament delivery device to emit a notification, change a setting on the handheld electronic computing device, silence a notification on the automated medicament delivery device, switch of an alert on the automated medicament delivery device, snooze an alert on the automated medicament delivery device, silence a notification on the handheld electronic computing device, issue an alarm, provide a response to a notification.

Embodiment 44: The automated medicament delivery device according to any of Embodiments 41 through 43, wherein the instructions, when executed, further cause the one or more processors to: maintain an input device in a low power mode until detection of the first tap; and switch the input device to a high-power mode in response to detecting the first tap.

Embodiment 45: The automated medicament delivery device according to any of Embodiments 41 through 44, wherein the high-power mode includes a higher sampling rate than the low power mode.

Embodiment 46: The automated medicament delivery device according to any of Embodiments 41 through 45, wherein the instructions, when executed, further cause the one or more processors to: maintain the input device in the high-power mode for a predetermined time; and in response to no subsequent tap being detected, switch the input device back to the low power mode.

Embodiment 47: The automated medicament delivery device according to any of Embodiments 41 through 46, wherein initiating the action includes sending a signal to a handheld electronic computing device associated with the automated medicament delivery device to initiate the action at the handheld electronic computing device.

Embodiment 48: A method, comprising: receiving, at a cloud server, an indication that an alarm has been triggered at an automated medicament delivery device; and notifying a caregiver that the alarm was triggered.

Embodiment 49: The method according to Embodiment 48, wherein the alarm is sent directly from the automated medicament delivery device.

Embodiment 50: The method according to any of Embodiments 48 and 49, wherein the alarm is chosen from among an indication of a fall of a patient, an indication of a recovery of the patient, an alarm associated with a health condition of the patient, an alarm triggered by the patient, and an alarm associated with a condition of the automated medicament delivery device.

Embodiment 51: The method according to any of Embodiments 48 through 50, wherein the alarm includes geographic coordinates appended thereto indicating a location where the alarm occurred.

Embodiment 52: The method according to any of Embodiments 48 through 51, wherein notifying the caregiver includes sending the notification to an electronic device of the caregiver from the cloud server.

Embodiment 53: The method according to any of Embodiments 48 through 52, wherein the notification is sent via at least one notification method chosen from among a push notification sent to an application operating on the electronic device of the caregiver, a message sent over a cellular network, a message sent via the internet, a phone call, a voice message, an email, and an Application Programming Interface notification.

Embodiment 54: The method according to any of Embodiments 48 through 53, further comprising: compiling alarms received at the cloud server and data associated therewith; analyzing the data for common occurrences of the alarms; and providing warnings to users about the common occurrences including relevant information for the common occurrences.

Embodiment 55: A cloud server, comprising: one or more processors; and memory including instructions, when executed, cause the one or more processors to: receive an indication that an alarm has been triggered at an automated medicament delivery device; and notify a caregiver that the alarm was triggered.

Embodiment 56: The cloud server according to Embodiment 55, wherein the alarm is received directly from the automated medicament delivery device.

Embodiment 57: The cloud server according to any of Embodiments 55 and 56, wherein the alarm is chosen from among an indication of a fall of a patient, an indication of a recovery of the patient, an alarm associated with a health condition of the patient, an alarm triggered by the patient, and an alarm associated with a condition of the automated medicament delivery device.

Embodiment 58: The cloud server according to any of Embodiments 55 through 57, wherein the alarm includes geographic coordinates appended thereto indicating a location where the alarm occurred.

Embodiment 59: The cloud server according to any of Embodiments 55 through 58, wherein notifying the caregiver includes sending the notification to an electronic device of the caregiver from the cloud server.

Embodiment 60: The cloud server according to any of Embodiments 55 through 59, wherein the notification is sent via at least one notification method chosen from among a push notification sent to an application operating on the electronic device of the caregiver, a message sent over a cellular network, a message sent via the internet, a phone call, a voice message, an email, and an Application Programming Interface notification.

Embodiment 61: The cloud server according to any of Embodiments 55 through 60, wherein the instructions, when executed, further cause the one or more processors to: compiling alarms received at the cloud server and data associated therewith; analyzing the data for common occurrences of the alarms; and providing warnings to users about the common occurrences including relevant information for the common occurrences.

Embodiment 62: A method, comprising: receiving, at a handheld electronic computing device associated with an automated medicament delivery device, an indication that an alarm has been triggered at the automated medicament delivery device; obtaining geographic coordinates associated with the alarm; and sending the indication that the alarm has been triggered and the geographic coordinates to a cloud server.

Embodiment 63: The method according to Embodiment 62, wherein the alarm is chosen from among an indication of a fall of a patient, an indication of a recovery of the patient, an alarm associated with a health condition of the patient, an alarm triggered by the patient, and a condition of the automated medicament delivery device.

Embodiment 64: The method according to any of Embodiments 62 and 63, wherein the geographic coordinates are obtained from one of a GPS sensor of the handheld electronic computing device at a time the indication is received at the handheld electronic computing device and the automated medicament delivery device at a time the alarm is triggered.

Embodiment 65: A handheld electronic computing device associated with an automated medicament delivery device, the handheld electronic computing device comprising: one or more processors; and memory including instructions, when executed, cause the one or more processors to: receive an indication that an alarm has been triggered at an automated medicament delivery device; obtain geographic coordinates associated with the alarm; and send the indication that the alarm has been triggered and the geographic coordinates to a cloud server.

Embodiment 66: The handheld electronic computing device according to Embodiment 65, wherein the alarm is chosen from among an indication of a fall of a patient, an indication of a recovery of the patient, an alarm associated with a health condition of the patient, an alarm triggered by the patient, and a condition of the automated medicament delivery device.

Embodiment 67: The handheld electronic computing device according to any of Embodiments 65 and 66, wherein the geographic coordinates are obtained from one of a GPS sensor of the handheld electronic computing device at a time the indication is received at the handheld electronic computing device and the automated medicament delivery device at a time the alarm is triggered.

Embodiment 68: A process, comprising: obtaining motion data by sampling a motion sensor of an automated medicament delivery device; determining a magnitude of dynamic motion data by subtracting a value of gravity from the motion data; determining a signal energy from the magnitude of dynamic motion data; determining whether an activity of a patient has occurred based at least in part by comparing the signal energy to an activity threshold value; and triggering an activity alert in response to a determination that the activity of the patient has occurred.

Embodiment 69: The process according to Embodiment 68, wherein the signal energy is an integral of the magnitude of the motion data.

Embodiment 70: The process according to any of Embodiments 68 and 69, further comprising: determining a pose of the patient using the magnitude of the dynamic motion data; and obtaining a time of day, and wherein determining whether the activity of the patient has occurred is based in part on the pose of the patient and the time of day.

Embodiment 71: The process according to any of Embodiments 68 through 70, wherein the activity is chosen from among exercise, inactivity, and sleep, and wherein: exercise is determined by comparing the signal energy to an exercise threshold value, and in response to the signal energy being above an exercise threshold value, indicating that the patient is exercising; inactivity is determined by comparing the signal energy to an inactivity threshold value, determining that the signal energy has been below the inactivity threshold value for a predetermined time threshold, and in response to the signal energy being below the inactivity threshold value for the predetermined time threshold, the pose being different than a sleeping posture, and the time of day being daytime, indicating that the patient is inactive; and sleep is determined by comparing the signal energy to the inactivity threshold value, determining that the signal energy has been below the inactivity threshold value for a predetermined time threshold, and in response to the signal energy being below the inactivity threshold value for the predetermined time threshold, the pose being that of a sleeping posture, and the time of day being nighttime, indicating that the patient is sleeping.

Embodiment 72: The process according to any of Embodiments 68 through 71, wherein the activity is exercise, and in response to determining that exercise occurred, perform at least one of set an activity mode in a control of medicament administration and set an alarm for a reminder to a patient wearing the automated medicament delivery device.

Embodiment 73: A device, comprising: one or more processors; and memory including instructions, when executed, cause the one or more processors to: obtain motion data by sampling a motion sensor of an automated medicament delivery device; determine a magnitude of dynamic motion data by subtracting a value of gravity from the motion data; determine whether an activity of a patient has occurred based at least in part by comparing signal energy to an activity threshold value; and trigger an activity alert in response to a determination that the activity of the patient has occurred.

Embodiment 74: The device according to Embodiment 73, wherein the signal energy is an integral of the magnitude of the motion data.

Embodiment 75: The device according to any of Embodiments 73 and 74, wherein the instructions, when executed, further cause the one or more processors to: determine a pose of the patient using the magnitude of the dynamic motion data; and obtain a time of day, and wherein determining whether the activity of the patient has occurred is based in part on the pose of the patient and the time of day.

Embodiment 76: The device according to any of Embodiments 73 through 75, wherein the activity is chosen from among exercise, inactivity, and sleep, and wherein: exercise is determined by comparing the signal energy to an exercise threshold value, and in response to the signal energy being above an exercise threshold value, indicating that the patient is exercising; inactivity is determined by comparing the signal energy to an inactivity threshold value, determining that the signal energy has been below the inactivity threshold value for a predetermined time threshold, and in response to the signal energy being below the inactivity threshold value for the predetermined time threshold, the pose being different than a sleeping posture, and the time of day being daytime, indicating that the patient is inactive; and sleep is determined by comparing the signal energy to the inactivity threshold value, determining that the signal energy has been below the inactivity threshold value for a predetermined time threshold, and in response to the signal energy being below the inactivity threshold value for the predetermined time threshold, the pose being that of a sleeping posture, and the time of day being nighttime, indicating that the patient is sleeping.

Embodiment 77: The device according to any of Embodiments 73 through 76, wherein the device is a handheld electronic computing device associated with the automated medicament delivery device, the activity is exercise, and in response to determining that exercise occurred, perform at least one of set an activity mode in a control of medicament administration and set an alarm for a reminder to the patient wearing the automated medicament delivery device.

Embodiment 78: A method, comprising: selecting an alert for implementation on an automated medicament delivery device; setting a time for the alert; selecting an alert type; and sending instructions to the automated medicament delivery device for implementation of the alert thereon.

Embodiment 79: The method according to Embodiment 78, wherein the alert type is selected from an audio pattern, a vibration pattern, and a pattern with a combination of audio and vibration patterns output by the automated medicament delivery device.

Embodiment 80: The method according to any of Embodiments 78 and 79, further comprising: receiving inputs defining a combination of alert outputs to define a custom pattern for the alert type; generating the custom pattern; and causing the custom pattern to output at the automated medicament delivery device.

Embodiment 81: A handheld electronic computing device associated with an automated medicament delivery device, the handheld electronic computing device comprising: one or more processors; and memory including instructions, when executed, cause the one or more processors to: select an alert for implementation on the automated medicament delivery device; setting a time for the alert; selecting an alert type; and sending instructions to the automated medicament delivery device for implementation of the alert thereon.

Embodiment 82: The handheld electronic computing device according to Embodiment 81, wherein the alert type is selected from an audio pattern, a vibration pattern, and a pattern with a combination of audio and vibration patterns output by the automated medicament delivery device.

Embodiment 83: The handheld electronic computing device according to any of Embodiments 81 and 82, wherein the instructions, when executed, further cause the one or more processors to: receive inputs defining a combination of alert outputs to define a custom pattern for the alert type; generate the custom pattern; and cause the custom pattern to output at the automated medicament delivery device.

**Claims**

1. A method, comprising:

   obtaining motion data by sampling a motion sensor of an automated medicament delivery device at a predetermined frequency;
   determining a magnitude of resultant values using the motion data;
   storing the resultant values in a buffer for a time window in a memory;
   determining a difference between a maximum value and a minimum value of the resultant values currently stored in the buffer;
   comparing the difference to a fall threshold value; and
   setting a fall indication at least partially responsive to the comparison.

2. The method of claim 1, wherein setting a fall indication at least partially responsive to the comparison comprises:
   setting the fall indication to a first value at least partially responsive to the difference being greater than the fall threshold value.

3. The method of claim 1 or 2, wherein the fall threshold value is at least 2 times any of an acceleration of gravity, a force of gravity, and 100 degrees per second.

4. The method of claims 1 to 3, further comprising sending an alert to at least one party, in particular to at least one party chosen from among a caregiver and an emergency service that a fall has been detected.

5. The method of claim 4, further comprising appending geographic coordinates indicating a location of the fall to the alert.

6. The method of claims 1 to 5, further comprising increasing the predetermined frequency in response to a determination of an increased fall risk.

7. The method of claims 1 to 6, further comprising setting the fall threshold value based in part on a location of the automated medicament delivery device on a user-body.

8. The method of claims 1 to 7, further comprising:

   in response to the indication that a fall has occurred, comparing the difference to a post fall threshold value; and
   in response to the difference being greater than the post fall threshold value, indicating that a patient has possibly recovered.

9. An automated medicament delivery device, comprising:

   a motion sensor;
   one or more processors; and
   memory including instructions, when executed, cause the one or more processors to:

   obtain motion data by sampling the motion sensor at a predetermined frequency;
   determine a magnitude of resultant values using the motion data;
   store the resultant values in in a buffer for a time window in the memory;
   determine a difference between a maximum value and a minimum value of the resultant values currently stored in the buffer;
   compare the difference to a fall threshold value; and
   in response to the difference being greater than the fall threshold value, indicate that a fall has been detected.

10. The automated medicament delivery device of claim 9, wherein the motion sensor includes at least one sensor chosen from among an accelerometer, a gyroscope, and an inertial measurement unit, and wherein the motion data includes data chosen from among acceleration data, angular velocity data, and a combination of acceleration data and angular velocity data.

11. The automated medicament delivery device of claim 9 or 10, wherein the resultant values are determined by combining the magnitude of the resultant value in each direction of a coordinate system.

12. The automated medicament delivery device of claims 9 to 11, wherein indicating that the fall has been detected includes sending a signal to a handheld electronic computing device associated with the automated medicament delivery device that the fall has been detected.

13. The automated medicament delivery device of claims 9 to 12, wherein the signal includes data related to the fall including the motion data and the resultant values.

14. The automated medicament delivery device of claims 9 to 13, wherein the instructions, when executed, further cause the one or more processors to increase the predetermined frequency in response to a determination of an increased fall risk.

15. The automated medicament delivery device of claims 9 to 14, wherein the instructions, when executed, further cause the one or more processors to set the fall threshold value based in part on a location of the automated medicament delivery device on a user-body.

100

ONE OR MORE
SERVICES 150

NETWORK 152

AUTOMATED MEDICAMENT
DELIVERY SYSTEM 114

118

124

128

132

120

122

126

130

116

106

ANALYTE SENSOR 102

110

108

112

104

HANDHELD ELECTRONIC
COMPUTING DEVICE 138

140

148

142

144

146

FIG. 1

**FIG. 2**

**FIG. 3**

400

OBTAIN MOTION DATA BY SAMPLING THE MOTION SENSOR AT A PREDETERMINED FREQUENCY ⟩~402

DETERMINE A MAGNITUDE OF RESULTANT VALUES USING THE MOTION DATA ⟩~404

STORE THE RESULTANT VALUES IN IN A BUFFER FOR A TIME WINDOW IN THE MEMORY ⟩~406

DETERMINE A DIFFERENCE BETWEEN A MAXIMUM VALUE AND A MINIMUM VALUE OF THE RESULTANT VALUES CURRENTLY STORED IN THE BUFFER ⟩~408

COMPARE THE DIFFERENCE TO A FALL THRESHOLD VALUE ⟩~410

SETTING A FALL INDICATION AT LEAST PARTIALLY RESPONSIVE TO THE DIFFERENCE BEING DETERMINED ⟩~412

SETTING THE FALL INDICATION TO A FIRST VALUE AT LEAST PARTIALLY RESPONSIVE TO THE DIFFERENCE BEING GREATER THAN THE FALL THRESHOLD VALUE ⟩~414

SETTING THE FALL INDICATION TO A SECOND VALUE AT LEAST PARTIALLY RESPONSIVE TO THE DIFFERENCE BEING LESS THAN OR EQUAL TO THE FALL THRESHOLD VALUE, THE SECOND VALUE DIFFERENT THAN THE FIRST VALUE ⟩~416

**FIG. 4**

500

IN RESPONSE TO AN INDICATION THAT A FALL HAS OCCURRED, OBTAIN MOTION DATA BY SAMPLING THE MOTION SENSOR AT A PREDETERMINED FREQUENCY — 502

DETERMINE A MAGNITUDE OF RESULTANT VALUES USING THE MOTION DATA — 504

STORE THE RESULTANT VALUES IN IN A BUFFER IN THE MEMORY — 506

DETERMINE A DIFFERENCE BETWEEN A MAXIMUM VALUE AND A MINIMUM VALUE OF THE RESULTANT VALUES CURRENTLY STORED IN THE BUFFER — 508

COMPARE THE DIFFERENCE TO A POST FALL THRESHOLD VALUE — 510

IN RESPONSE TO THE DIFFERENCE BEING GREATER THAN THE POST FALL THRESHOLD VALUE, INDICATE THAT THE PATIENT HAS POSSIBLY RECOVERED — 512

**FIG. 5**

600

| DETECT A FIRST TAP | 602 |

| LISTEN FOR ONE OR MORE TAP SEQUENCES IN RESPONSE TO DETECTING THE FIRST TAP | 604 |

| INITIATE AN ACTION IN RESPONSE TO RECOGNITION OF A TAP SEQUENCE ASSOCIATED WITH THE ACTION | 606 |

**FIG. 6**

700

| RECEIVE, AT A CLOUD SERVER, AN INDICATION THAT AN ALARM HAS BEEN TRIGGERED AT AN AUTOMATED MEDICAMENT DELIVERY DEVICE | 702 |

| NOTIFY A CAREGIVER THAT THE ALARM WAS TRIGGERED | 704 |

**FIG. 7**

800

RECEIVE, AT A CONTROLLER, AN INDICATION THAT AN ALARM HAS BEEN TRIGGERED AT AN AUTOMATED MEDICAMENT DELIVERY DEVICE — 802

OBTAIN GEOGRAPHIC COORDINATES ASSOCIATED WITH THE ALARM — 804

SEND THE INDICATION THAT THE ALARM HAS BEEN TRIGGERED AND THE GEOGRAPHIC COORDINATES TO A CLOUD SERVER — 806

**FIG. 8**

900

908
POSE DETERMINATION

TIME OF DAY — 910

902
MOTION DATA

GRAVITY SUBTRACTION

904

914
TRIGGER ACTIVITY ALLERT

ACTIVITY CONFIRMED?

YES

912

SIGNAL ENERGY

906

**FIG. 9**

1000

SELECT AN ALERT TO CUSTOMIZE ——1002

SET A TIME FOR THE ALERT ——1004

SELECT AN ALERT TYPE ——1006

SEND INSTRUCTIONS TO THE AUTOMATED MEDICAMENT DELIVERY DEVICE FOR IMPLEMENTATION OF THE ALERT THEREON ——1008

**FIG. 10**

Europäisches
Patentamt

European
Patent Office

Office européen
des brevets

# EUROPEAN SEARCH REPORT

**Application Number**

EP 24 20 8665

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CN 111 657 918 A (UNIV ELECTRONIC SCI & TECH CHINA) 15 September 2020 (2020-09-15) | 1-5,8-13 | INV.<br>G16H50/30 |
| A | * claim 1-2;<br>the whole document * | 6,7,14,<br>15 | G16H50/70 |
| | - - - - - | | |
| Y | CN 113 813 471 A (JIANGSU MAODU YUNKE MEDICAL TECH CO LTD)<br>21 December 2021 (2021-12-21) | 1-5,8-13 | |
| A | * claim 9;<br>the whole document * | 6,7,14,<br>15 | |
| | - - - - - | | |
| A | ANTONIO SANTOYO-RAMÓN JOSÉ ET AL: "A study of the influence of the sensor sampling frequency on the performance of wearable fall detectors",<br>MEASUREMENT, INSTITUTE OF MEASUREMENT AND CONTROL. LONDON, GB,<br>vol. 193, 1 March 2022 (2022-03-01),<br>XP087007976,<br>ISSN: 0263-2241, DOI:<br>10.1016/J.MEASUREMENT.2022.110945<br>[retrieved on 2022-03-01]<br>* abstract * | 1-15 | |
| | - - - - - | | |

**TECHNICAL FIELDS SEARCHED (IPC)**

G16H

The present search report has been drawn up for all claims

| Place of search | Date of completion of the search | Examiner |
|---|---|---|
| Munich | 12 March 2025 | Bankwitz, Robert |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 20 8665

This annex lists the patent family members relating to the patent documents cited in the above-mentioned European search report.
The members are as contained in the European Patent Office EDP file on
The European Patent Office is in no way liable for these particulars which are merely given for the purpose of information.

12-03-2025

| Patent document cited in search report | Publication date | Patent family member(s) | Publication date |
|---|---|---|---|
| CN 111657918 A | 15-09-2020 | NONE | |
| CN 113813471 A | 21-12-2021 | NONE | |

EPO FORM P0459

For more details about this annex : see Official Journal of the European Patent Office, No. 12/82